# FASCICULE DE BREVET EUROPEEN

(11) **EP 0 693 963 B1**
(45) Date de publication et mention de la délivrance du brevet: **26.03.1997**
(21) Numéro de dépôt: 94913638.6
(22) Date de dépôt: 13.04.1994
(51) Int. Cl.: B01J 13/14

(54) **UTILISATION D'UNE REACTION DE TRANSACYLATION ENTRE UN POLYSACCHARIDE ESTERIFIE ET UNE POLYAMINE POUR FORMER EN MILIEU AQUEUX UNE MEMBRANE AU MOINS EN SURFACE DE PARTICULES GELIFIEES, PARTICULES AINSI REALISEES, PROCEDES ET COMPOSITIONS EN CONTENANT**
VERWENDUNG EINER TRANSACYLIERUNGSREAKTION, ZWISCHEN EINEM VERESTERTEN POLYSACCHARID UND EINEM POLYAMIN, UM IM WÄSSERIGEM MEDIUM WENIGSTENS AN DER OBERFLAECHE AUS GELIERTEN PARTIKELN EINE MEMBRAN ZU FORMEN,SO HERGESTELLTE PARTIKEL,DIESE ENTHALTENDE ZUSAMMENSETZUNGEN UND VERFAHREN
USE OF A TRANSACYLATION REACTION BETWEEN AN ESTERIFIED POLYSACCHARIDE AND A POLYAMINE TO FORM A MEMBRANE AT LEAST ON THE SURFACE OF GELLED PARTICLES IN AN AQUEOUS MEDIUM, RESULTING PARTICLES, PREPARATION METHODS THEREFOR AND COMPOSITIONS CONTAINING SAME

(30) Priorité: 13.04.1993 FR 9304332
(43) Date de publication de la demande: 31.01.1996
(73) Titulaire: COLETICA, 69007 Lyon (FR)
(72) Inventeur: LEVY, Marie-Christine, F-51100 Reims (FR); EDWARDS-LEVY, Florence, F-51100 Reims (FR); ORLY, Isabelle, F-69002 Lyon (FR)
(74) Mandataire: Portal, Gérard
(86) Numéro de dépôt international: FR9400409
(87) Numéro de publication internationale: WO9423832

(56) Documents cités:
- GB-A- 2 145 992
- US-A- 3 503 769
- US-A- 4 352 883
- US-A- 5 132 242

## Description

La présente invention concerne essentiellement l'utilisation d'une réaction de transacylation avec formation de liaisons covalentes amide entre un polysaccharide estérifié et une polyamine pour former en milieu aqueux une membrane stable au moins en surface de particules gélifiées, les particules ainsi réalisées, leurs procédés de fabrication ainsi que les compositions en contenant.

Plus précisément, la présente invention concerne essentiellement l'utilisation d'une réaction de transacylation entre d'une part un polysaccharide porteur de groupements carboxyliques estérifiés, et d'autre part une polyamine, pour former en milieu aqueux une membrane stable au moins en surface de particules gélifiées, les particules modifiées ainsi réalisées, les procédés utilisés pour former de telles particules modifiées, et les compositions contenant les particules ainsi obtenues, telles que compositions cosmétiques, pharmaceutiques, thérapeutiques, agroalimentaires, enzymatiques, biotechnologiques, de réactifs ou de diagnostic.

Il est bien connu que l'on peut préparer très facilement des sphères par exemple à partir d'alginate de sodium, en utilisant la propriété que présentent les solutions d'alginate de gélifier en présence de cations tels que par exemple les ions calcium. Le matériau à encapsuler dans les sphères est tout d'abord dispersé dans la solution aqueuse d'alginate. Cette solution est ajoutée goutte à goutte à une solution aqueuse d'un sel de calcium. Il y a gélification immédiate, qui produit des sphères d'alginate gélifié. La surface des sphères peut être ensuite stabilisée par immersion dans une solution d'un polymère polycationique tel que la poly-L-lysine ou la polyéthylèneimine (Lim, F., US- A-4, 352, 883, 1982). Il se forme une membrane en périphérie résultant de l'association ionique entre l'alginate et le polycation. Cette membrane laisse passer les petites molécules tout en retenant les grosses molécules et les cellules. Il est possible ensuite de liquéfier le gel interne en immergeant les sphères d'alginate stabilisées par le polymère polycationique, dans une solution de citrate, de manière à chélater le calcium dans les sphères (Lim, F., US Patent, 4, 352, 883, 1982). Le matériau incorporé reste alors enfermé à l'intérieur de la membrane.

Ce procédé présente le grand avantage de s'effectuer entièrement en milieu aqueux et de conserver une excellente viabilité aux cellules vivantes encapsulées, qui peuvent se multiplier à l'intérieur de la capsule. Aussi est-il largement utilisé pour l'inclusion de microorganismes, de cellules et de tissus vivants. Ainsi des sphères d'alginate contenant des microorganismes sont utilisées pour réaliser des fermentations dans l'industrie alimentaire (fermentation des produits laitiers, de la bière, du champagne.....). Ces techniques sont appliquées à des cellules ou organes végétaux ou animaux dans un but de cryoprotection ou de production de métabolites, à des cellules ou tissus animaux (îlots de Langerhans, hépatocytes....) pour implantation en médecine humaine ou vétérinaire (thérapie cellulaire), ou pour effectuer des tests de toxicologie in vitro. Des cellules sont également cultivées dans de telles sphères pour la production de substances biologiques telles que les anticorps monoclonaux élaborés par les hybridomes, qui s'accumulent dans les sphères et sont ainsi aisément récoltées après ouverture des membranes.

Toutefois, le procédé présente des inconvénients liés à la nature de la membrane. Comme elle n'implique pas de liaisons covalentes mais uniquement des liaisons ioniques entre l'alginate et un polycation, elle est d'une stabilité limitée (Dupuy et al., J. Biomed. Mat. Res., 1988, 22, 1061-1070). Les polymères ont tendance à la longue à passer en solution. Par ailleurs, si la pression augmente à l'intérieur de la capsule sous l'effet de la multiplication cellulaire, la membrane ne peut résister à la pression et il y a relargage de cellules dans le milieu. Il est donc souvent nécessaire d'appliquer successivement des couches alternées d'alginate puis de polycation ("sandwiches") pour obtenir une membrane solide, qui ne laisse pas diffuser le contenu (Wong H. and Chang T.M.S. Biomat. Art. Cells & Immob. Biotech., 1991, 19, 675-686).

La document Demande Françise FR-A-2 694 894 décrit l'utilisation d'une réaction de transacylation entre un ester polysaccharidique, tel que l'alginate de propylène glycol (PGA) et une substance polyaminée, telle qu'une diamine ou une protéine, pour fabriquer des microcapsules. La réaction de transacylation entre l'ester et la polyamine est déclenchée en milieu alcalin et produit une membrane formée d'un polysaccharide associé à une polyamine par des liaisons amide. Ce document décrit plusieurs procédés de préparation de microcapsules, tous mettant en jeu une étape d'émulsification, au cours de laquelle soit une phase aqueuse est dispersée dans une phase hydrophobe, soit une phase hydrophobe est dispersée dans une phase aqueuse. Dans tous les cas, les microcapsules s'individualisent à partir de l'émulsion initiale, par alcalinisation de l'émulsion.

Si on tente d'appliquer directement cette réaction de transacylation à une suspension aqueuse de sphères formées d'un polysaccharide gélifiant sous forme gélifiée par un agent de gélification tel qu'un cation mono ou polyvalent, on ne parvient pas à créer un membrane autour des sphères. Ainsi par exemple, si on prépare des sphères en ajoutant goutte à goutte une solution d'alginate de sodium à une solution aqueuse calcique, si ensuite on disperse lesdites sphères dans une solution aqueuse renfermant un polysaccharide estérifié, tel que l'alginate de propylène glycol, et une protéine, et si enfin on alcalinise la suspension aqueuse de manière à déclencher la réaction de transacylation entre le polysaccharide estérifié et la protéine, on observe une prise en masse de la solution aqueuse dès que le pH est suffisamment élevé pour permettre la réaction.

La présente invention a pour but de créer, en milieu aqueux, une membrane stable, impliquant des liaisons covalentes, au moins en surface de sphères individualisées par gélification d'un polysaccharide ou d'une polyamine en particulier par un cation mono ou polyvalent, comme par exemple par un sel de calcium.

La présente invention a encore pour but de préparer des particules, stables, à partir de polyamines notamment de protéines, à température du laboratoire, en milieu aqueux, sans agent réticulant bifonctionnel, et renfermant soit une substance hydrosoluble, soit une mousse de bulles d'air, soit une substance insoluble dans l'eau, soit un matériau vivant tel que des cellules, des tissus ou des organes animaux ou végétaux, ou des graines ou des oeufs, ou des microorganismes, soit une phase hydrophobe, selon que l'on préparera au départ, soit une solution, soit une mousse, soit une suspension, soit une émulsion dans la solution gélifiable initiale.

La présente invention a également pour but de préparer des particules stables, à partir de protéines, en limitant les altérations de leur structure, de manière à obtenir une biocompatibilité améliorée, et dans le cas où la protéine est douée d'une activité biologique spécifique, de manière à préserver cette activité.

La présente invention a également pour but de préparer des particules renfermant des matériels vivants en ménageant la viabilité des matériels encapsulés.

La présente invention a pour but d'utiliser la réaction de transacylation entre un polysaccharide estérifié et une polyamine pour fabriquer des particules sphériques entourées d'une membrane, en milieu strictement aqueux.

La présente invention a encore pour but d'appliquer la réaction de transacylation entre un polysaccharide estérifié et une polyamine à des sphères formées d'un polysaccharide gélifiant ou d'une polyamine gélifiante sous forme gélifiée par un agent de gélification tel qu'un cation mono ou polyvalent, ou un polyphosphate, ou une solution de pH >6,2 selon la nature du composé gélifiant, de manière à fournir soit des sphères à membrane externe et contenu gélifié, soit des sphères à membrane externe et contenu liquide, soit des sphères constituées d'un gel rigidifié dans toute sa masse.

La présente invention a encore pour but de résoudre les problèmes techniques ci-dessus énoncés, avec l'utilisation de procédés de fabrication simples, utilisables à l'échelle industrielle et permettant en outre de règler la taille des particules, en particulier dans une plage de dimension allant de quelques micromètres à 10 millimètres.

Selon la présente invention, il a été découvert de manière parfaitement inattendue, que la création d'une membrane stable impliquant des liaisons covalentes, au moins en surface de sphères formées d'un polysaccharide gélifiant ou d'une polyamine gélifiante sous forme gélifiée par un agent de gélification, pouvait être réalisée de manière extrêmement simple par exemple par dissolution d'un polysaccharide estérifié et d'une polyamine dans la solution initiale d'un polysaccharide gélifiant tel que l'alginate de sodium. La solution obtenue est ajoutée sous forme de gouttes à un bain gélifiant tel que par exemple une solution d'un sel de calcium, de manière à individualiser les particules par gélification. Les particules sont lavées à l'eau puis redispersées dans l'eau. Il suffit alors d'alcaliniser la suspension aqueuse pour déclencher immédiatement la réaction de transacylation entre le polysaccharide estérifié et la polyamine incorporés aux sphères. On peut aussi disperser directement les sphères dans une solution aqueuse alcaline, ou encore alcaliniser directement le bain de gélification après la formation des particules gélifiées. On constate alors qu'une membrane se forme au moins à la périphérie des sphères. Après réaction, la suspension est neutralisée au moyen d'un acide et les particules sont lavées à l'eau. On obtient alors des sphères formées du polysaccharide gélifiant sous forme gélifiée, entourées au moins en surface d'une membrane constituée d'une polyamine directement associée par des liaisons amide au polysaccharide porteur de carboxyles initialement estérifiés.

Il a été également découvert que l'on pouvait créer une membrane stable au moins en surface de sphères formées du polysaccharide gélifiant sous forme gélifiée, par exemple d'alginate gélifié par un cation polyvalent, tel que par exemple un sel de calcium, en incorporant seulement le polysaccharide estérifié à la solution initiale du polysaccharide gélifiant. Les sphères sont individualisées par gélification, par exemple au contact d'un sel de calcium, lavées, puis dispersées dans une solution aqueuse renfermant la polyamine. On déclenche alors la réaction par alcalinisation de la suspension. On peut aussi disperser directement les sphères dans une solution aqueuse alcaline de la polyamine, ou encore ajouter directement la solution alcaline de polyamine au bain de gélification. Après réaction, on neutralise la suspension par addition d'un acide.

Dans une variante, le protocole ci-dessus est appliqué en incorporant la polyamine à la fois à la phase aqueuse initiale gélifiable et à la phase externe à alcaliniser. On compense ainsi les pertes éventuelles de polyamine par diffusion hors des particules ou billes en milieu aqueux.

Il a été encore découvert que, dans le cas où le polysaccharide estérifié est lui-même capable de gélifier en présence de cations, comme c'est le cas par exemple pour l'alginate de propylène-glycol ou les pectines au contact de sels de calcium, il est possible alors de ne pas ajouter de polysacharide gélifiant à la solution aqueuse initiale à gélifier contenant la polyamine et lé polysaccharide estérifié. Le polysaccharide estérifié est alors employé à des concentrations plus élevées de manière à pouvoir jouer un double rôle dans cette variante: assurer la formation des sphères par gélification d'une part, intervenir dans la formation ultérieure de la membrane par transacylation lors de l'alcalinisation ultérieure de la suspension de sphères d'autre part.

On a également découvert que le contenu gélifié des particules ainsi entourées d'une membrane selon les principes ci-dessus, pouvait être liquéfié par immersion dans une solution de citrate ou de phosphate. Après rinçage des particules, on obtient des sphères dont la membrane parfaitement visible emprisonne un contenu liquide.

Enfin on a découvert que dans ces systèmes, on pouvait aisément contrôler l'épaisseur de la membrane, sa biodégradabilité et l'étroitesse de ses pores par variation de la durée de la réaction de transacylation et/ou par variation des conditions d'alcalinisation de la phase aqueuse au cours de l'étape de transacylation. La membrane est d'autant plus épaisse, et d'autant moins sensible à la lyse protéasique et présentant des pores d'autant plus étroits que la réaction est plus prolongée et/ou que la quantité d'agent alcalin ajoutée à la suspension aqueuse de sphères à enrober, ou à la phase aqueuse utilisée pour leur dispersion ou encore au bain de gélification, est plus élevée. Les quantités d'acide à ajouter sont alors augmentées également, de manière à permettre la neutralisation de la suspension. Si l'alcalinisation est réalisée avec des quantités d'agent alcalin encore plus élevées et/ou avec des temps de réaction encore plus prolongés, la réaction de transacylation concerne alors la totalité de la masse de la sphère dont le contenu est ainsi rigidifié et garde sa consistance solide même après traitement au citrate de sodium.

C'est sur la base de cette découverte totalement inattendue pour l'homme de l'art, que la présente invention a été réalisée.

L'invention représente un progrès déterminant pour l'homme de l'art, compte tenu de ce que les membranes des sphérules obtenues résultent de l'établissement de liaisons covalentes amide, par la réaction de transacylation. Elles sont ainsi parfaitement stables. De plus, leur constitution ne fait intervenir que des substances biocompatibles. Elles pourront donc trouver de nombreuses applications dans divers domaines tels que la pharmacie, la cosmétique, le domaine biomédical, l'industrie alimentaire et les biotechnologies.

En outre, la réaction de transacylation pouvant se limiter à la couche superficielle des sphères, le procédé permet l'incorporation de substances fragiles y compris des produits biologiques, des cellules ou tissus végétaux, et des cellules, tissus animaux ou des groupes de tissus ou des organes vivants, ou encore de microorganismes, sans altération.

Enfin, la possibilité de faire varier l'épaisseur de la membrane offre l'avantage de permettre un ajustement des propriétés de la membrane. Par exemple, si les sphères contiennent une substance active dans le domaine de la cosmétologie, on pourra choisir des conditions telles que la membrane présente une résistance mécanique faible, de sorte qu'elle se détruise lors de l'application sur la peau en libérant son contenu. Ou bien au contraire, on pourra choisir que la membrane résiste et joue le rôle de réservoir à libération prolongée permettant la diffusion lente du contenu in situ. Dans ce cas, les variations d'épaisseur de la membrane permettront également de moduler la cinétique de diffusion du contenu dans le milieu extérieur. En outre, l'épaisseur de la membrane conditionnera sa sensibilité à la lyse enzymatique. Ainsi, dans le domaine pharmaceutique, on pourra préparer des billes, capsules, microcapsules, sphères ou microsphères permettant l'administration de principes actifs par diverses voies telles que la voie orale, la voie parentérale, la voie rectale ou la voie topique. C'est ainsi par exemple que l'on pourra préparer des capsules gastrorésistantes dont l'entérosolubilité sera modulable, ce qui permettra d'administrer par la voie orale des principes actifs susceptibles d'être dégradés par l'estomac ou des substances irritantes pour la muqueuse gastrique. Ces substances seront libérées plus ou moins rapidement dans l'intestin, selon que la membrane sera plus ou moins rapidement dégradée par les protéases intestinales. Par ailleurs, les variations d'épaisseur des membranes et le choix de la protéine peuvent permettre d'obtenir des variations dans leur transparence. Selon les cas on pourra préparer des membranes complètement transparentes ou translucides ou opaques. Des membranes opaques pourront être préférées par exemple si le contenu est altérable à la lumière.

Enfin, par variations de la durée de la réaction de transacylation et/ou du pH de ladite réaction, on pourra moduler la porosité de la membrane et ainsi ajuster cette porosité en fonction du poids moléculaire de la substance que l'on désire retenir à l'intérieur des particules ou bien faire diffuser à l'extérieur lors de leur utilisation. Cette possibilité constitue un avantage important des procédés selon l'invention tant pour des applications in vivo, par exemple basées sur la libération d'une substance telle qu'une hormone dans l'organisme, que pour des applications in vitro, en particulier pour la production de substances biologiques par des cellules, tissus ou microorganismes génétiquement modifiés ou non.

Ainsi, selon un premier aspect, la présente invention a pour objet l'utilisation d'une réaction de transacylation avec formation de liaisons covalentes amide entre au moins un polysaccharide porteur de groupements carboxyliques estérifiés et au moins une substance polyaminée pour la fabrication en milieu aqueux de particules, notamment billes, capsules, microcapsules, sphères, microsphères, comprenant au moins en surface une membrane formée par le produit de réaction selon ladite réaction de transacylation avec formation de liaisons covalentes amide entre ledit polysaccharide estérifié et ladite polyamine, ledit polysaccharide estérifié et ladite polyamine étant plus particulièrement définis à partir de la description suivante dans son ensemble, ainsi que par les revendications. De préférence, ces particules contiennent un principe actif cosmétique, pharmaceutique, ou une substance d'intérêt agro-alimentaire, ou une protéine douée d'une activité biologique telle qu'une enzyme, une hormone, un anticorps ou l'hémoglobine, ou des particules insolubles telles que des particules de charbon actif, ou une mousse de bulles d'air, ou une phase liquide aqueuse ou hydrophobe, ou un vaccin, ou des cellules, tissus, ou organes vivants animaux ou végétaux, ou des oeufs ou des graines, ou des microorganismes tels que des bactéries ou des levures, ou des constituants cellulaires tels que des microsomes hépatiques, ou des gamètes, des embryons, du matériel génétique du règne animal ou du règne végétal.

Selon un deuxième aspect, la présente invention a également pour objet des particules, notamment billes, capsules, microcapsules, sphères, microsphères, caractérisées en ce qu'elles comprennent au moins un polysaccharide estérifié et au moins une polyamine, additionnés d'au moins un polysaccharide gélifiable lorsque ni le polysaccharide estérifié ni la polyamine ne sont gélifiables dans les conditions opératoires choisies, ladite particule comprenant au moins en surface une membrane formée par le produit de la réaction de transacylation avec formation de liaisons covalentes amide entre le polysaccharide estérifié et ladite polyamine au sein d'un gel éventuellement liquéfiable.

Selon une variante de réalisation, le polysaccharide estérifié précité est un polysaccharide porteur de groupements carboxyliques estérifiés, en particulier un alginate de propylène glycol, une pectine, en particulier une pectine fortement méthoxylée, ou tout autre composé obtenu par estérification de carboxyles de polysaccharides porteurs de carboxyles.

Selon une autre variante de réalisation, la polyamine précitée comprend une protéine, un polypeptide, un polyaminoacide, un polysaccharide porteur de groupements aminés tels que le chitosan, une substance organique, aliphatique, alicyclique ou aromatique, porteuse de plusieurs groupements aminés primaires ou secondaires, telle que l'éthylènediamine, l'hexaméthylènediamine, la pipérazine, la phénylènediamine ou la polyéthylène imine.

Selon une autre variante de réalisation, le polysaccharide précité est un polysaccharide gélifiable, en particulier choisi parmi le groupe consistant d'un alginate, d'un carraghénane, en particulier le kappa-carraghénane, une pectine gélifiable, en particulier une pectine faiblement méthoxylée, le chitosan.

D'autres variantes de réalisation sont mentionnées dans la description et les revendications.

Selon une variante de réalisation, les particules présentent, à l'intérieur de la membrane, un contenu solide formé du constituant gélifiable sous forme gélifiée notamment par un cation mono ou polyvalent, tel qu'un sel de calcium, dans lequel est dissous ou dispersé le matériau encapsulé.

Selon une autre variante de réalisation, les particules sont caractérisées en ce qu'elles présentent, à l'intérieur de la membrane, un contenu liquéfié, du fait d'un traitement ultérieur de liquéfaction du gel interne des sphères par exemple par le citrate ou le phosphate de sodium, pouvant contenir des substances en solution, suspension ou émulsion.

Selon une autre variante de réalisation, les particules sont caractérisées en ce qu'elles présentent à l'intérieur de la membrane un contenu liquide constitué d'un liquide aqueux ou hydrophobe, introduit dans les particules par coextrusion laminaire de la solution aqueuse à gélifier et du liquide à encapsuler.

Avantageusement, les proportions de polysaccharide gélifiant par rapport au polysaccharide estérifié peuvent varier de 0 % à 300 % en poids. Les proportions de polysaccharide estérifié par rapport à la polyamine peuvent varier de 3 % à 500 % en poids.

Selon un mode de réalisation particulièrement avantageux, ces particules, en particulier ces billes, capsules, microcapsules, sphères, microsphères, contiennent un principe actif en particulier choisi parmi le groupe consistant d'un principe actif cosmétique, pharmaceutique, une substance d'intérêt agro-alimentaire, une substance intéressant le diagnostic ou les réactifs, une protéine douée d'une activité biologique telle qu'une enzyme, une hormone, un anticorps, ou l'hémoglobine, ou une mousse de bulles d'air, ou des particules insolubles telles que des particules de charbon actif, ou une phase liquide hydrophobe, ou un vaccin, ou des cellules, tissus, ou organes vivants, du règne animal ou végétal, des embryons, des oeufs, ou des graines éventuellement additionnées de substances diverses, ou des microorganismes tels que des bactéries ou des levures, ou des constituants cellulaires tels que des microsomes hépatiques, ou des gamètes, des embryons, du matériel génétique du règne animal ou du règne végétal.

Selon une autre variante de réalisation avantageuse de l'invention, les particules sont caractérisées en ce qu'elles présentent une partie centrale constituée d'une phase aqueuse éventuellement gélifiée ou liquéfiée, contenant un matériel en particulier un matériel vivant tel que des microorganismes comme des levures ou des bactéries, ou des cellules vivantes, en particulier des cellules végétales ou des tissus végétaux, ou des cellules ou des tissus animaux, ou une substance biologique comme par exemple une enzyme, et une couche externe ne contenant pas ledit matériel et comportant au moins en surface une membrane formée par le produit de la réaction de transacylation avec formation de liaisons covalentes amide entre un polysaccharide estérifié et au moins une polyamine. De telles particules peuvent être par exemple préparées par coextrusion laminaire de deux phases aqueuses de composition différente, comme cela sera décrit plus loin en particulier en référence au septième aspect de l'invention.

Cette constitution particulière permet d'enfermer les matériels inclus dans la partie centrale. On pourra ainsi notamment éviter tout relargage dudit matériel, en particulier de microorganismes ou de cellules, dans le milieu d'utilisation. Ceci est particulièrement important dans les procédés de fermentation, et notamment dans les procédés de fermentation ou de refermentation pour la préparation de champagne. En outre, dans le cas de particules, en particulier de billes, destinées à être implantées pour thérapie cellulaire ou pour thérapie enzymatique, la couche externe empêche tout développement de réaction immunologique en isolant les cellules ou les substances biologiques du milieu extérieur.

Selon un troisième aspect, la présente invention concerne un procédé de fabrication de particules, en particulier de billes, capsules, microcapsules, sphères ou microsphères, caractérisé par les étapes successives suivantes:
a) on prépare une première solution aqueuse initiale gélifiable par un agent de gélification contenant au moins un polysaccharide estérifié et au moins une polyamine, additionnés d'au moins un polysaccharide gélifiable, lorsque ni le polysaccharide estérifié, ni la polyamine ne sont gélifiables dans les conditions opératoires choisies.
b) on fait tomber des gouttes de cette première solution dans une deuxième solution aqueuse formant bain de gélification, contenant un agent de gélification, de manière à individualiser des particules par gélification à l'aide dudit agent de gélification.
c) On met ensuite en contact les particules gélifiées obtenues avec une solution aqueuse alcaline, de manière à déclencher la réaction de transacylation, au moins à la surface des particules gélifiées entre le polysaccharide estérifié et la polyamine contenus dans lesdites particules, pendant une période de temps prédéterminée, pour former une membrane au moins en surface desdites particules.
d) on ajoute au milieu réactionnel un agent acide de manière à neutraliser et ainsi stabiliser les particules, en obtenant ainsi des particules comprenant au moins en surface une membrane formée par le produit de réaction avec formation de liaisons covalentes entre le polysaccharide estérifié et la polyamine.

En variante, on peut alcaliniser le bain de gélification, ou bien on sépare les particules du bain de gélification, on les lave à l'eau puis on les disperse dans une solution alcaline, ou bien on les disperse dans l'eau puis on alcalinise la suspension.

Dans une variante, le protocole ci-dessus est appliqué en incorporant la polyamine à la fois à la phase aqueuse initiale gélifiable et à la phase externe alcalinisée. On peut ainsi compenser les pertes éventuelles de polyamine par diffusion hors des billes en milieu aqueux.

Selon un quatrième aspect, la présente invention concerne un procédé de fabrication de particules, caractérisé par les étapes successives suivantes:
a) on prépare une première solution aqueuse initiale gélifiable par un agent de gélification contenant au moins un polysaccharide estérifié et au moins une polyamine, additionnée d'au moins un polysaccharide gélifiable lorsque le polysaccharide estérifié n'est pas gélifiable dans les conditions opératoires choisies.
b) on fait tomber des gouttes de cette première solution dans une deuxième solution aqueuse formant bain de gélification contenant un agent de gélification, de manière à individualiser des particules par gélification à l'aide dudit agent de gélification.
c) on met en contact les particules gélifiées obtenues avec une solution aqueuse alcaline contenant une polyamine, de manière à déclencher une réaction dite de transacylation à la surface des particules gélifiées, entre le polysaccharide estérifié contenu dans lesdites particules et la polyamine, pendant une période de temps prédéterminée, pour former une membrane au moins en surface desdites particules,
d) on ajoute au milieu réactionnel un agent acide de manière à neutraliser et ainsi stabiliser les particules, en obtenant ainsi des particules comprenant au moins en surface une membrane formée par le produit de réaction avec formation de liaisons covalentes entre le polysaccharide estérifié et la polyamine et renfermant une goutte du liquide à encapsuler précité.

Avantageusement, les procédés précités pourront utiliser, pour former les gouttes de solution gélifiable, tout moyen permettant d'individualiser des gouttes tel que par exemple une seringue munie d'une aiguille ou une pompe péristaltique équipée d'un tube muni d'une aiguille ou un système de dispersion par utilisation d'un pulvérisateur à air comprimé, ou encore en coupant mécaniquement à l'aide d'un vibreur un écoulement laminaire réalisé par extrusion à travers une buse.

Selon un cinquième aspect, la présente invention fournit encore un procédé de fabrication de particules, en particulier billes, capsules, microcapsules, sphères ou microsphères, ou contenant une phase liquide encapsulée, aqueuse ou hydrophobe, caractérisé en ce qu'il comprend les étapes successives suivantes:
a) on prépare une première solution aqueuse initiale gélifiable par un agent de gélification contenant au moins un polysaccharide estérifié et au moins une polyamine, additionnés d'au moins un polysaccharide gélifiable lorsque ni le polysaccharide estérifié, ni la polyamine ne sont gélifiables dans les conditions opératoires choisies;
b) on prépare une phase liquide, aqueuse ou hydrophobe à encapsuler;
c) on réalise à travers une buse d'extrusion une coextrusion laminaire de ladite première solution aqueuse en flux externe et de la phase liquide aqueuse ou hydrophobe à encapsuler en flux interne, dans des conditions réalisant une dissociation de l'écoulement laminaire en gouttelettes individuelles, par exemple en soumettant l'écoulement laminaire à des vibrations.
d) on fait tomber les gouttelettes individuelles dans une deuxième solution aqueuse contenant un agent de gélification de manière à individualiser des particules par gélification à l'aide dudit agent de gélification .
e) on met en contact les particules gélifiées obtenues avec une solution aqueuse alcaline de manière à déclencher la réaction de transacylation au moins à la surface des particules entre le polysaccharide estérifié et la polyamine pendant une période de temps prédéterminée pour former une membrane au moins en surface desdites particules.
f) on ajoute au milieu réactionnel un agent acide de manière à neutraliser et ainsi stabiliser les particules, en obtenant ainsi des particules comprenant au moins en surface une membrane formée par le produit de réaction avec formation de liaisons covalentes entre le polysaccharide estérifié et la polyamine et renfermant une goutte du liquide à encapsuler précité.

Selon un sixième aspect, la présente invention fournit encore un procédé de fabrication de particules en particulier de billes, capsules, microcapsules, sphères ou microsphères, contenant un matériel encapsulé présent dans l'étape b) du procédé selon le cinquième aspect, on prépare une phase aqueuse à encapsuler renfermant éventuellement un polysaccharide gélifiable et contenant un matériel, en particulier un matériel vivant tel que des microorganismes comme des levures ou des bactéries, ou des cellules ou des tissus vivants, animaux ou végétaux, ou une substance biologique comme par exemple une enzyme.

Selon un septième aspect, la présente invention fournit encore un procédé de fabrication de particules en particulier de billes, capsules, microcapsules, sphères ou microsphères, contenant un matériel encapsulé présent exclusivement dans sa partie centrale, et caractérisé en ce qu'il comprend les étapes successives suivantes :
a) on prépare une première solution aqueuse initiale gélifiable par un agent de gélification, contenant au moins un polysaccharide estérifié et au moins une polyamine additionnés d'au moins un polysaccharide gélifiable par ledit agent de gélification lorsque ni le polysaccharide estérifié ni la polyamine ne sont gélifiables dans les conditions opératoires choisies.
b) on prépare une phase aqueuse à encapsuler renfermant éventuellement au moins un polysaccharide gélifiable et contenant un matériel, en particulier un matériel vivant tel que des microorganismes comme des levures ou des bactéries, ou des cellules ou tissus vivants, en particulier des cellules ou des tissus végétaux, des cellules ou tissus animaux, ou des groupes de tissus, ou une substance biologique comme par exemple une enzyme.
c) on réalise à travers une buse d'extrusion une co-extrusion laminaire de ladite première solution aqueuse initiale gélifiable en flux externe, et de la phase aqueuse à encapsuler en flux interne dans des conditions opératoires réalisant une dissociation d'écoulement laminaire en gouttelettes individuelles, par exemple en soumettant l'écoulement laminaire à des vibrations.
d) on fait tomber les gouttelettes individuelles dans une deuxième solution aqueuse contenant un agent de gélification de manière à individualiser des particules par gélification.
e) on met en contact les particules avec une solution aqueuse alcaline éventuellement additionnée d'au moins une polyamine, de manière à déclencher la réaction de transacylation dans la couche externe des particules entre ledit polysaccharide estérifié et ladite polyamine, pendant une période de temps prédéterminée, pour former une membrane au moins en surface des particules.
f) on ajoute au milieu réactionnel un agent acide de manière à neutraliser et ainsi stabiliser les particules.

Des appareils de mise en oeuvre de ce procédé de coextrusion sont bien connus à l'homme de l'art qui peut se reporter aux documents WO 92/06771 ou GB-A-2 192 171 ou encore EP-A-0 173 915.

Avantageusement, les procédés précités peuvent encore comprendre une étape complémentaire de séparation de particules par tout moyen approprié, notamment par décantation naturelle ou centrifugation, après avoir effectué un ou plusieurs lavages.

Selon une caractéristique avantageuse du procédé de fabrication selon l'invention, les particules, une fois séparées du milieu réactionnel, peuvent être placées dans une solution d'un agent liquéfiant en particulier de citrate ou de phosphate de sodium, de manière à liquéfier le gel interne. Les échanges entre le matériau encapsulé dans les particules et le milieu externe seront ainsi facilités. En outre, s'il s'agit d'un matériau biologique vivant, celui-ci pourra continuer à croître et se multiplier, à l'intérieur de la particule.

Selon une caractéristique avantageuse du procédé de fabrication selon l'invention, les particules peuvent être séchées, par exemple par lyophilisation, par nébulisation ou dans un séchoir à lit fluidisé, par exemple à air avantageusement chauffé à une température appropriée, ce qui permet d'obtenir un produit aisément manipulable de stockage facile et de bonne conservation.

Selon une autre caractéristique avantageuse du procédé de fabrication, les particules pourront être aisément produites dans des conditions d'asepsie, en opérant en atmosphère stérile et en utilisant des matières premières stérilisées, par exemple par radiostérilisation ou par filtration stérilisante.

Selon une autre caractéristique avantageuse du procédé de fabrication, les particules une fois fabriquées pourront être stérilisées par radiostérilisation. Ainsi elles seront utilisables par exemple pour l'administration de principes actifs par voie parentérale, en médecine humaine ou vétérinaire.

Selon une caractéristique avantageuse du procédé de fabrication, la polyamine est une protéine, de préférence choisie parmi les protéines hydrophiles ou traitées de manière à être rendues hydrophiles, c'est-à-dire solubles dans l'eau ou dispersibles dans l'eau, contenant des groupements aminés libres.

Il n'est pas essentiel que les matières protéiques utilisées dans la réaction soient des protéines pures. Elles peuvent être utilisées sous la forme de mélanges naturels ou non contenant une ou plusieurs protéines hydrophiles, comme par exemple le lait ou un concentré de protéines du lactosérum.

Des exemples de protéines qui peuvent être utilisées dans l'invention et qui remplissent les conditions qui consistent à être hydrophiles ou bien qui peuvent être traitées pour être hydrophiles, sont les albumines comme la sétumalbumine, l'ovalbumine, l'alpha-lactalbumine, des globulines, le fibrinogène, la caséine, des protéines végétales telles que les protéines du soja ou du blé, des gluténines qui de préférence auront été dégradées, des scléroprotéines solubilisées, le collagène, l'atélocollagène, la gélatine, les hydrolysats de gélatine, les peptones, l'hémoglobine, des enzymes telles que la catalase, la phosphatase alcaline, des hormones, des immunoglobulines ou des anticorps tels que les anticorps monoclonaux.

Comme exemples de mélanges contenant des protéines hydrophiles, on peut citer le lait entier ou écrémé totalement ou partiellement, le lait en poudre, le lait condensé, les protéines du lactosérum, l'oeuf entier, le blanc d'oeuf, le jaune d'oeuf, la farine de soja, les concentrés protéiques de soja, les préparations alimentaires liquides au soja, le lait de coco, les mélanges d'atélocollagène et de glycosaminoglycannes, le sérum sanguin, le bouillon de viande, les milieux de culture, en particulier les milieux pour culture de cellules végétales ou de tissus végétaux, de cellules animales ou de tissus animaux, ou les milieux pour culture de microorganismes.

Selon une caractéristique avantageuse du procédé de fabrication selon l'invention, la substance polyaminée est un polysaccharide porteur de groupements aminés, tel que par exemple le chitosan.

Selon une caractéristique avantageuse du procédé de fabrication selon l'invention, le polysaccharide estérifié est un polysaccharide hydrophile porteur de nombreux groupements carboxyliques, lesquels sont estérifiés dans une proportion au moins égale à 50 %, soit par modification chimique, soit naturellement. Selon une caractéristique préférée, le polysaccharide estérifié est choisi parmi l'alginate de propylène-glycol (PGA) et les pectines, de préférence choisie parmi les pectines fortement méthoxylées.

Selon une caractéristique avantageuse du procédé de fabrication selon l'invention, le polysaccharide gélifiable est choisi parmi un alginate, une pectine, en particulier une pectine faiblement méthoxylée, un carraghénane, en particulier un kappa-carraghénane, un polysaccharide porteur de groupements aminés de préférence gélifiable pour un pH supérieur à 6,2 ou en présence de polyphosphate, tel que le chitosan.

Selon une caractéristique avantageuse du procédé de fabrication selon l'invention, la concentration en polysaccharide gélifiant de la solution aqueuse initiale à disperser et gélifier, est comprise entre 0 et 20 %, de préférence de 1 % (p/v).

Selon une autre caractéristique avantageuse du procédé selon l'invention, la concentration en polysaccharide estérifié de la solution aqueuse initiale est comprise entre 0,5 % et 20 %, et encore de préférence voisine de 2 % p/v.

Selon une autre caractéristique avantageuse du procédé selon l'invention, la concentration en polyamine de la solution aqueuse initiale est comprise entre 1 % et 30 % p/v.

Selon une autre caractéristique avantageuse du procédé selon l'invention, la concentration du bain gélifiant en cation monovalent ou polyvalent en particulier en sel de calcium, ou en sel de potassium ou en polyphosphate, est comprise entre 0,01 M et 3 M, et de préférence 0,8 M à 1M.

Selon une autre caractéristique avantageuse du procédé selon l'invention, le temps pendant lequel les particules sont agitées au sein du bain gélifiant est compris entre 1 et 40 minutes.

Selon une autre caractéristique avantageuse du procédé selon l'invention, la quantité d'agent alcalin à ajouter pour déclencher la réaction de transacylation, à la suspension aqueuse des particules gélifiées d'alginate, ou à la phase aqueuse dans laquelle on disperse directement les sphères, est telle que le pH de la suspension aqueuse de sphères soit compris entre 8 et 14, et encore de préférence entre 10,5 et 12,5.

Selon une autre caractéristique avantageuse du procédé selon l'invention, la quantité d'agent acide à ajouter à la suspension alcaline de particules après la réaction de transacylation, est telle que l'eau de suspension des particules soit neutralisée ou amenée à un pH légèrement acide .

Selon une autre caractéristique avantageuse du procédé selon l'invention, l'agent alcalin à ajouter pour déclencher la réaction de transacylation est par exemple choisi parmi la soude, la potasse, l'ammoniaque ou un composé aminé tel que par exemple la triéthanolamine, la triéthylamine, la polyéthylèneimine.

Selon une autre caractéristique avantageuse du procédé selon l'invention, l'épaisseur de la membrane formée par la réaction de transacylation, et sa résistance à la lyse enzymatique, peuvent être augmentées par augmentation de la durée de ladite réaction de transacylation et/ou par des variations de la composition de la solution alcaline déclenchant ladite réaction de transacylation, et en particulier, par augmentation de la quantité d'agent alcalin utilisé pour préparer ladite solution alcaline.

Selon une autre caractéristique avantageuse du procédé selon l'invention, le temps pendant lequel les particules sont maintenues au sein de la solution alcaline pour que se développe la réaction de transacylation est compris entre 5 min et 1 heure, de préférence entre 5 min et 30 min, de préférence encore, il est de 15 min.

Selon une autre caractéristique avantageuse du procédé selon l'invention, l'agent acide utilisé pour neutraliser la suspension aqueuse de particules après la réaction de transacylation, est par exemple choisi parmi les acides organiques monocarboxyliques ou polycarboxyliques, porteurs ou non de fonctions alcool, comme l'acide acétique, l'acide citrique, l'acide tartrique, l'acide succinique, l'acide malique, l'acide lactique ou un acide minéral comme l'acide chlorhydrique ou l'acide sulfurique.

Selon une autre caractéristique avantageuse du procédé selon l'invention, le temps de neutralisation des particules, c'est-à-dire le temps d'agitation nécessaire après ajout de l'acide au milieu réactionnel est compris entre 5 min et 1 heure, de préférence entre 5 min et 30 min, de préférence encore, il est de 15 min.

Selon une autre caractéristique avantageuse du procédé selon l'invention, la concentration de la solution de citrate ou de phosphate à utiliser pour liquéfier le contenu des particules après formation de la membrane par transacylation est comprise entre 0,01 M et 1 M, de préférence elle est voisine de 0,2 à 0,5 M.

Selon une autre caractéristique avantageuse du procédé selon l'invention, le temps pendant lequel les particules, après la réaction de transacylation, doivent être agitées dans la solution de citrate ou de phosphate pour liquéfaction du contenu est compris entre 2 min et 30 min, de préférence il est de 10 min.

On peut introduire dans les particules diverses substances qui seront incorporées soit à la solution aqueuse initiale gélifiable des procédés décrits en troisième et quatrième aspect, soit à la phase liquide aqueuse ou hydrophobe à encapsuler par coextrusion selon les procédés décrits en cinquième, sixième et septième aspect.

Ainsi, on peut introduire dans la phase aqueuse initiale, ou dans la phase liquide à encapsuler par coextrusion, un ou plusieurs principes actifs, directement ou à l'état de solution, de suspension ou d'émulsion, en particulier une ou plusieurs substances d'intérêt cosmétique, pharmaceutique ou biomédical ou alimentaire. On peut par exemple émulsionner dans la phase aqueuse initiale une phase liquide hydrophobe, telle qu'une huile végétale, une huile minérale, une huile de silicone, une huile essentielle ou une solution huileuse d'une substance liposoluble. On peut incorporer directement la phase liquide hydrophobe en l'encapsulant par coextrusion.

On peut aussi disperser dans la solution aqueuse initiale, ou dans la phase liquide à encapsuler par coextrusion, des particules d'un matériau adsorbant tel que le charbon actif. De telles sphères contenant un matériau adsorbant enfermé dans une membrane ont des applications en particulier dans des circuits extracorporels d'épuration sanguine pour débarrasser le sang de ses métabolites toxiques (insuffisance rénale) ou de substances toxiques dans des cas d'intoxication. On peut également emprisonner une mousse à l'intérieur des particules. Ainsi par exemple, on peut incorporer des bulles de gaz tel que l'air à la solution initiale contenant l'alginate de sodium, le polysaccharide estérifié et la protéine, en la soumettant à une agitation très vive. On applique ensuite à la mousse le procédé de l'invention en la faisant tomber en gouttes dans le bain gélifiant, puis en formant une membrane autour des particules gélifiées, par réaction de transacylation. Après séchage, les particules contiennent une multitude de bulles de gaz emprisonnées. De telles particules trouvent une indication d'emploi dans les méthodes de diagnostic médical par échographie. En outre, leur faible densité leur permet de flotter à la surface de l'eau, ce qui laisse envisager d'autres applications telles que la préparation de formes à durée de séjour gastrique prolongé pour permettre une libération prolongée d'un principe actif en amont des sites de résorption intestinaux. Une autre application intéressante de particules dégradables flottantes à membrane est la préparation de sphérules à disperser à la surface des eaux et renfermant une substance toxique pour les larves de moustiques. La libération lente du produit en surface de l'eau permet une grande efficacité du traitement avec moins de produit car elle cible les larves situées à ce niveau.

On peut également disperser dans la phase aqueuse initiale, ou dans la phase liquide à encapsuler par coextrusion, un vaccin pour relargage lent in situ, ou encore des matériels vivants appartenant au règne animal ou au règne végétal.

Des exemples de matériels vivants qui peuvent être incorporés dans les particules selon l'invention sont des microorganismes pour réaliser des synthèses ou des bioconversions, tels que des bactéries comme celles utilisées pour les fermentations de produits laitiers, ou celles utilisées pour la dépollution des eaux, ou des champignons tels que les mycorhizes ou les levures comme par exemple les levures utilisées dans la fabrication de la bière, ou les levures utilisées pour la prise de mousse du champagne, ou des micro-algues, des graines éventuellement additionnées de substances diverses protectrices ou influençant leur germination ou leur croissance, des embryons somatiques végétaux pour produire des semences synthétiques, des apex végétaux, des cellules ou des tissus végétaux notamment pour effectuer des biosynthèses ou des bioconversions, du matériel vivant appartenant au règne animal, tel que des cellules ou des tissus utilisables notamment pour effectuer des tests de toxicologie in vitro comme les cellules hépatiques, les chondrocytes, les neurones, des cellules ou des groupes de cellules ou des tissus utilisables dans le cadre d'une thérapie cellulaire comme les îlots de Langerhans pour le traitement du diabète, les cellules de la médullosurrénale ou les cellules chromaffines pour le traitement de la maladie de Parkinson ou pour le traitement des douleurs chroniques, ou des cellules utilisables pour la production de diverses substances biologiques telles que des hormones, des enzymes, des facteurs de croissance, de l'interféron, des facteurs de coagulation, ou des hybridomes pour production d'anticorps monoclonaux, ou des constituants cellulaires tels que les microsomes hépatiques pour effectuer des bioconversions, ou des oeufs, ou des gamètes, des embryons ou du matériel génétique appartenant au règne animal ou au règne végétal.

Lorqu'on utilise pour réagir avec le polysaccharide estérifié une protéine douée d'une activité biologique spécifique telle qu'une enzyme ou l'hémoglobine, les particules obtenues selon l'invention peuvent constituer une forme immobilisée d'utilisation facile, notamment dans les domaines des biotechnologies, des bioréactifs ou de la thérapeutique. Ainsi par exemple, les particules préparées à partir d'enzymes ont des indications d'emploi en thérapie substitutive dans les déficiences enzymatiques, ou dans les systèmes extra-corporels d'épuration sanguine, ou pour catalyser des réactions en biotechnologie. Les particules préparées à partir d'hémoglobine ont des applications comme hématies artificielles ou encore en biotechnologie pour l'oxygénation des bioréacteurs.

Enfin, selon un huitième aspect, la présente invention concerne encore une composition telle qu'une composition cosmétique, ou une composition pharmaceutique, ou une composition alimentaire, ou une composition enzymatique, ou une composition pour thérapie cellulaire, ou une composition pour thérapie enzymatique, ou une composition pour épuration sanguine, ou une composition pour diagnostic ou pour réactifs, ou une composition pour tests toxicologiques in vitro, ou une composition pour graines enrobées, ou une composition pour production biotechnologique, caractérisée en ce qu'elle comprend des particules, notamment billes, capsules, microcapsules, sphères, microsphères, comprenant au moins en surface une membrane formée par le produit de réaction, selon ladite réaction de transacylation, avec formation de liaisons covalentes entre au moins une polyamine et au moins un polysaccharide porteur de groupements carboxyliques estérifiés.

D'autres buts, cactéristiques et avantages de l'invention apparaîtront clairement à la lumière de la description explicative qui va suivre, faite en référence à plusieurs exemples de réalisation de l'invention donnés simplement à titre d'illustration et qui ne sauraient donc en aucune façon limiter la portée de l'invention, et en référence aux figures annexées dans lesquelles:
- la Figure 1 représente un cliché obtenu en microscopie électronique à balayage de capsule de l'invention selon l'exemple 26, montrant des fibroblastes humains tapissant la paroi interne de la membrane de la capsule.
- la Figure 2 représente un appareil de fabrication de particules selon l'invention notamment sous forme de billes, capsules, microcapsules, sphères ou microsphères, par coextrusion laminaire, en référence à l'exemple 29.
- La Figure 3 présente, en µmol de paranitrophénol libéré en fonction du temps (en minutes), les résultats du dosage d'activité enzymatique de la phosphatase alcaline libre, utilisée comme témoin dans l'exemple 23 selon l'invention.
- La Figure 4 présente, en µmol de paranitrophénol libéré en fonction du temps (en minutes), les résultats du dosage d'activité enzymatique des billes de phosphatase alcaline de l'invention préparées selon l'exemple 23.

### Exemple 1 selon l'invention

Fabrication de sphères à contenu solide avec membrane formée à partir de sétumalbumine humaine (HSA) et d'alginate de propylène glycol (PGA).

### a) Préparation de la solution aqueuse initiale

On prépare, par agitation magnétique à température ambiante, 12 ml d'une solution dans l'eau distillée renfermant 2% d'un PGA présentant un taux d'estérification compris entre 80 et 85% (Kelcoloïd S (r), KELCO International), 1% d'alginate de sodium (Manucol DH, KELCO) et 5% d'HSA (Centre de Transfusion Sanguine, Strasbourg).

### b) Individualisation des sphères par traitement au chlorure de calcium

A l'aide d'une pompe péristaltique Gilson équipée d'un tube Tygon muni d'une aiguille, on fait tomber environ 10 ml de cette solution goutte à goutte dans 50 ml d'une solution de CaCl2 à 10% sous agitation magnétique. On maintient l'agitation pendant 10 min, puis les sphères formées sont rincées plusieurs fois à l'eau distillée.

### c) Formation de la membrane

Les sphères sont remises en suspension dans 50 ml d'eau distillée sous agitation magnétique.
- On ajoute 400 µl de NaOH 1N, et on laisse agiter 15 min.
- On ajoute 150 µl de HCl 1N, et on laisse agiter 15 min.

Les sphères à membrane obtenues sont rincées plusieurs fois à l'eau distillée. On obtient des sphères pleines lisses et opalescentes, de diamètre environ 4 mm, présentant une membrane externe bien visible. A l'état de suspension aqueuse, elles peuvent être conservées plus de 3 semaines aussi bien à +4°C qu'à l'étuve à 45°C. Ces sphères sont intactes après lyophilisation et se réhydratent rapidement. Elles peuvent être radiostérilisées (par rayons gamma, 25 kGy) sans altération.

### Essai de tolérance après implantation sous-cutanée chez l'animal

Ces essais ont été effectués sur quatre rats anesthésiés, dont le dos a été rasé. Deux incisions latérales ont été pratiquées sur chaque animal, par lesquelles ont été introduites, d'un côté, 3 à 4 sphères lyophilisées telles quelles, de l'autre côté 3 à 4 sphères lyophilisées préalablement réhydratées dans du soluté isotonique de NaCl stérile. Les incisions ont été suturées. Aucune réaction inflammatoire importante n'a été constatée chez les animaux, qui n'ont pas manifesté d'oedème, ni développé d'infection. Un examen des sites d'implantation après sacrifice 24 h, 3 jours, une semaine et 2 semaines après l'implantation, n'a pas révélé de réaction tissulaire anormale. En outre l'examen a montré la résorption des sphères, bien amorcée après une semaine, très avancée après 2 semaines.

### Exemple 2 selon l'invention

Fabrication de particules à contenu liquide, à membrane formée à partir de sérumalbumine humaine (HSA) et d'alginate de propylène glycol (PGA).

Le protocole décrit à l'exemple 1 est reproduit. Les particules à membranes obtenues subissent alors un traitement destiné à liquéfier le gel interne:

### Traitement au citrate de sodium:

Le lot de particules est remis en suspension dans 50 ml d'une solution aqueuse de citrate de sodium à 10% et agité pendant 10 min. Ensuite les particules liquéfiées sont rincées plusieurs fois à l'eau distillée.

On obtient des sphères transparentes, de diamètre voisin de 3,3 mm, formées d'un contenu liquide emprisonné dans une fine membrane, souple et incolore. Après lyophilisation, la fine membrane est souvent cassée.

### Essais de stabilité des particules conservées à l'état de suspension aqueuse

- Un lot de particules est conservé à l'état de suspension dans l'eau distillée à la température de +4°C. Aucune dégradation des particules n'est observée après 6 mois de conservation.
- Un lot de particules est mis en suspension dans l'eau distillée, puis placé dans une étuve à la température de 45°C. Aucune dégradation n'a été observée après 3 semaines.

### Essais de dégradation in vitro par les protéases

Protocole: Un échantillon de 20 particules est placé dans un tube à essais contenant:
- soit une solution de pepsine ( from porcine stomach mucosa, SIGMA) à pH 1,2 (milieu gastrique artificiel, USP XXI)
- soit une solution de trypsine (from porcine pancreas, type II, SIGMA) à 0,5%, dans un tampon de pH 7,5.

Une agitation magnétique est installée dans les tubes, qui sont placés au bain-marie à 37°C. L'essai est reproduit trois fois.
Résultats: (Moyenne des trois essais)

Les particules résistent à la pepsine pendant plus de 24 H, tandis qu'il n'y a plus aucune capsule intacte après une incubation de 130 min en présence de trypsine.

### Exemple 3 selon l'invention

Le protocole décrit à l'exemple 2 est appliqué en utilisant, au cours du stade de formation de la membrane:
- 800 µl de NaOH 1 N pour l'alcalinisation
- puis 300 µl de HCl 1N pour la neutralisation.

Les vésicules obtenues après liquéfaction du gel interne présentent une paroi nettement plus épaisse que celles de l'exemple 2 obtenues en utilisant deux fois moins de soude. Après lyophilisation, la membrane est intacte, et, placée en présence d'eau se réhydrate en 15 min.

### Essais de dégradation enzymatique

- Pepsine: aucune dégradation après 24 h.
- Trypsine: le temps au bout duquel il n'y a plus une particule intacte est prolongé à 175 min.

### Exemple 4 selon l'invention

Le protocole décrit à l'exemple 2 est appliqué en utilisant, au cours du stade de formation de la membrane:
- 1200 µl de NaOH 1 N pour l'alcalinisation
- puis 450 µl de HCl 1N pour la neutralisation.

La membrane des particules obtenues est très épaisse, plus épaisse et moins élastique que celle des capsules de l'exemple 3. Après lyophilisation, la réhydratation des vésicules est plus lente (90 min).

### Essais de dégradation enzymatique

- Pepsine: aucune dégradation après 24 h.
- Trypsine: le temps au bout duquel il n'y a plus une particule intacte est prolongé à 215 min.

### Exemple 5 selon l'invention

Le protocole décrit à l'exemple 2 est appliqué en utilisant, au cours du stade de formation de la membrane:
- 1600 µl de NaOH 1 N pour l'alcalinisation
- puis 600 µl de HCl 1N pour la neutralisation.

Les particules ne présentent pratiquement plus de contenu liquide, la transacylation concernant dans ce cas des couches plus profondes des particules. Après lyophilisation, la réhydratation des sphères est obtenue après 1h.

### Essais de dégradation enzymatique

- Pepsine: aucune dégradation après 24 h.
- Trypsine: le temps au bout duquel il n'y a plus une particule intacte est prolongé à 250 min.

Les observations des particules obtenues selon les protocoles des exemples 2, 3, 4 et 5 montrent bien que l'on peut moduler l'épaisseur de la membrane et faire varier sa sensibilité à la lyse protéasique, en faisant varier les volumes de soude ajoutés pour déclencher la réaction de transacylation.

### Exemple 6 selon l'invention

Fabrication de sphères à membrane formée à partir d'HSA et de PGA, et renfermant des particules de charbon actif.

Le protocole décrit à l'exemple 3 est appliqué en dispersant dans la phase aqueuse initiale, 600 mg de particules de charbon actif (granulométrie de 125 à 315 µm) et en utilisant une seringue pour ajouter cette suspension à la solution calcique. On obtient des sphères de couleur noire, de taille voisine de 4 mm, dans lesquelles on distingue les particules de charbon. Ces particules sont intactes après lyophilisation.

### Exemple 7 selon l'invention

Fabrication aseptique de particules à membrane formée à partir d'HSA et de PGA.

Le protocole décrit à l'exemple 3 est reproduit, en utilisant des matières premières (alginate de sodium, HSA, et PGA) stérilisées par rayons gamma (25 kGy) et de l'eau stérile pour préparations injectables. La fabrication est réalisée sous flux laminaire. On obtient des sphères régulières entourées d'une membrane qui apparaît parfaitement bien formée après le traitement de liquéfaction au citrate de sodium.

### Exemple 8 selon l'invention

Fabrication de particules à membrane formée à partir d'HSA et de PGA et contenant des bulles d'air.

Le protocole décrit à l'exemple 3 est appliqué en ajoutant un stade d'incorporation de bulles d'air à la phase aqueuse initiale et en utilisant une seringue pour ajouter la mousse ainsi obtenue à la solution calcique.

A cet effet, la solution initiale renfermant l'alginate de sodium, le PGA et l'HSA est soumise à une agitation mécanique à 2000 rpm pendant 5 min, ce qui produit une mousse. La mousse est ensuite ajoutée goutte à goutte à la solution calcique sous agitation magnétique, et les opérations suivantes sont effectuées comme décrit à l'exemple 3. Ensuite, après formation de la membrane par alcalinisation puis neutralisation par acidification de la suspension de particules, les particules recouvertes d'une membrane sont rincées plusieurs fois à l'eau distillée et remises en suspension dans l'eau distillée. La suspension est ensuite divisée en deux parties. La moitié du lot, séparée par centrifugation est remise en suspension dans 25 ml de solution de citrate de sodium à 10 % et agitée pendant 10 min. Puis les sphères liquéfiées sont rincées plusieurs fois à l'eau distillée. L'autre partie du lot est conservée en suspension aqueuse.

Les particules non citratées flottent sur l'eau, sont sphériques, blanches opaques, dures, et on aperçoit les bulles d'air en périphérie. Elles sont intactes après lyophilisation, se réhydratent lentement et flottent sur l'eau. Après liquéfaction au citrate et regonflement dans l'eau, le diamètre des particules augmente et on voit nettement la membrane formée d'une mousse rigidifiée.

### Exemple 9 selon l'invention

Fabrication de particules à membrane formée à partir d'ovalbumine et de PGA.

Le protocole décrit à l'exemple 1 est appliqué, en remplaçant l'HSA par de l'ovalbumine (OSI), en augmentant à 8 % la concentration de protéine, et en doublant toutes les quantités de réactifs.

Après formation de la membrane et neutralisation par HCl de la suspension de particules, celle-ci est divisée en deux parties:
- l'une subit le traitement de liquéfaction au citrate de sodium, tel que décrit à l'exemple 2,
- l'autre ne subit pas de traitement de liquéfaction.

Les particules non traitées au citrate ont une forme ovoïde et un aspect opalescent. A l'état de suspension aqueuse, elles peuvent être conservées plus de 3 semaines aussi bien à +4°C qu'à l'étuve à 45°C. Elles sont intactes après lyophilisation, et se réhydratent rapidement. Les particules traitées au citrate sont parfaitement sphériques et translucides. A l'état de suspension aqueuse, elles peuvent être conservées plus de 3 semaines aussi bien à +4°C qu'à l'étuve à 45°C. Beaucoup sont cassées après le traitement de lyophilisation.

### Exemple 10 selon l'invention

Le protocole décrit à l'exemple 9 est reproduit en utilisant un volume double de solution de soude et de solution acide.

Tandis que les particules d'alginate gélifié se présentent, avant le traitement d'alcalinisation, comme des billes ovoïdes, beiges, translucides, elles deviennent, après alcalinisation de la suspension puis neutralisation, beaucoup plus opaques, et de consistance plus dure. Après traitement de liquéfaction au citrate, les particules deviennent translucides et molles. Elles présentent une paroi épaisse, et reprennent progressivement une forme sphérique après séjour dans l'eau distillée. Elles résistent parfaitement au traitement de lyophilisation et se réhydratent en 5 à 10 min.

### Exemple 11 selon l'invention

Le protocole décrit à l'exemple 9 est reproduit en utilisant un volume triple de solution de soude et de solution acide.

Avant liquéfaction au citrate, les particules, opaques, présentent un paroi plus épaisse et sont de consistance plus dure que les particules de l'exemple 10. Après liquéfaction au citrate, elles deviennent plus translucides et plus molles. Elles résistent parfaitement à la lyophilisation et se réhydratent en 1h 30 environ.

### Exemple 12 selon l'invention

Fabrication de particules à membrane formée à partir d'ovalbumine et de PGA et contenant de l'huile d'olive.

Le protocole décrit à l'exemple 9 est reproduit en émulsionnant dans la solution aqueuse initiale 4,8 ml d'huile d'olive par agitation mécanique à 5000 rpm pendant 5 min. On obtient après formation de la membrane et acidification de la suspension, des billes blanches, flottant sur l'eau. Des gouttelettes d'huile sont visibles dans les sphères au microscope. Après liquéfaction du gel interne par le citrate, les billes sont plus molles et plus plates. Elles redeviennent sphériques après quelques heures de séjour dans l'eau distillée. Le centre des particules se présente sous forme d'une émulsion fluide.

### Exemple 13 selon l'invention

Fabrication de particules à membrane formée à partir d'ovalbumine et de PGA et contenant de l'huile essentielle de menthe poivrée.

Le protocole décrit à l'exemple 12 est reproduit en remplaçant l'huile d'olive par de l'huile essentielle de menthe poivrée.

L'aspect des particules obtenues avant et après traitement au citrate est comparable à celui des particules préparées selon l'exemple 12.

### Exemple 14 selon l'invention

Fabrication de particules à membrane formée à partir d'ovalbumine et de pectine.

Le protocole décrit à l'exemple 10 est reproduit en remplaçant le PGA par la pectine de pomme (FLUKA, estérification 70 à 75%).

Les particules traitées au citrate de sodium comme les particules non traitées sont semblables aux particules préparées à partir de PGA selon l'exemple 10. Après lyophilisation, les sphères sont intactes avec une membrane bien visible. La réhydratation est rapide (10 min).

### Exemple 15 selon l'invention

Fabrication de particules à membrane formée à partir de sérumalbumine bovine (BSA) et de PGA.

Le protocole décrit à l'exemple 10 est reproduit en remplaçant l'ovalbumine par la sérumalbumine bovine (fraction V, SIGMA) utilisée à la concentration de 2 %. Les particules ont des caractéristiques comparables à celles des particules préparées selon l'exemple 10. Après traitement au citrate, les particules peuvent être conservées à l'état de suspension aqueuse plus de 3 semaines, aussi bien à +4°C qu'à l'étuve à 45°C.

### Exemple 16 selon l'invention

Fabrication de particules à membrane formée à partir d'hémoglobine et de PGA .

Le protocole décrit à l'exemple 10 est reproduit en remplaçant l'ovalbumine par l'hémoglobine bovine (SIGMA) utilisée à la concentration de 10 %. Les particules ont des caractéristiques comparables à celles des particules préparées selon l'exemple 10. Après traitement au citrate, les particules peuvent être conservées à l'état de suspension aqueuse plus de 3 semaines, aussi bien à +4°C qu'à l'étuve à 45°C.

### Exemple 17 selon l'invention

Fabrication de particules à membrane formée à partir de protéines du lactosérum et de PGA .

Le protocole décrit à l'exemple 10 est reproduit en remplaçant l'ovalbumine par un concentré de protéines du lactosérum (Prosobel S65E, Bel Industries) utilisée à la concentration de 5 %.

Les particules ont des caractéristiques comparables à celles des particules préparées selon l'exemple 10. Après traitement au citrate, les particules peuvent être conservées à l'état de suspension aqueuse plus de 3 semaines, aussi bien à +4°C qu'à l'étuve à 45°C.

### Exemple 18 selon l'invention

Fabrication de particules à membrane formée à partir d'un hydrolysat de gélatine et de PGA .

Le protocole décrit à l'exemple 10 est reproduit en remplaçant l'ovalbumine par un hydrolysat de gélatine, soluble dans l'eau à froid (gélatine atomisée DSF, Méro-Rousselot-Satia) utilisée à la concentration de 10 %.

Les particules ont des caractéristiques comparables à celles des particules préparées selon l'exemple 10. Après traitement au citrate, les particules peuvent être conservées à l'état de suspension aqueuse plus de 3 semaines, aussi bien à +4°C qu'à l'étuve à 45°C.

### Exemple 19 selon l'invention

Fabrication de particules à membrane formée à partir de protéines du soja et de PGA.

Le protocole décrit à l'exemple 10 est reproduit en remplaçant l'ovalbumine par la farine de soja (type I: not roasted, SIGMA). Pour préparer la phase aqueuse, on procède alors comme suit: on ajoute à 20 ml d'eau distillée 10 % de farine de soja. Après 15 min d'agitation magnétique, le milieu est centrifugé pendant 3 min à 5000 rpm. Dans 12 ml du surnageant, on dissout 1 % d'alginate de sodium et 2 % de PGA.

Les particules ont des caractéristiques comparables à celles des particules préparées selon l'exemple 10. Après traitement au citrate, les particules peuvent être conservées à l'état de suspension aqueuse plus de 3 semaines, aussi bien à +4°C qu'à l'étuve à 45°C.

### Exemple 20 selon l'invention

Fabrication de particules à membrane formée à partir d'ovalbumine et de PGA et contenant du rouge de phénol.

Le protocole décrit à l'exemple 9 est reproduit en diminuant de moitié les quantités de réactifs, en dissolvant 6 mg de rouge de phénol dans la solution initiale contenant l'alginate, le PGA et l'ovalbumine et en supprimant le stade de liquéfaction. La solution initiale est jaune d'or.

Après addition de la solution de soude qui déclenche la réaction de transacylation, on observe les particules au microscope. Durant les 15 min. qui suivent l'addition, il n'y a pas de modification de la couleur jaune du coeur des particules. En revanche, la couche superficielle est colorée en rose fuschia, ce qui indique l'alcalinisation de la périphérie du gel. Cette observation est confirmée par un examen des particules après incision de la partie périphérique.

Cette expérience avec un colorant indicateur de pH montre que, dans les conditions de l'essai, la réaction de transacylation est localisée dans la couche superficielle des sphères, puisque les variations de pH n'en atteignent pas le centre. La réaction, bien localisée, peut donc être appliquée à l'encapsulation de cellules et autres matériaux vivants.

### Exemple 21 selon l'invention

Fabrication de particules à membrane formée à partir d'ovalbumine et de PGA et contenant des bactéries lactiques.

L'expérience a été effectuée en utilisant des bactéries lactiques lyophilisées, Lactobacillus casei var. rhamnosus, présentées en gélules de 250 mg, renfermant au minimum 8. 108 germes / gramme (Antibiophilus (r) , Lyocentre).

### * Encapsulation des bactéries

Dans 12 ml l'eau distillée stérile, on dissout:
- 240 mg de PGA
- 120 mg d'alginate de sodium
- 720 mg d'ovalbumine

On disperse dans cette solution 240 mg de poudre de bactéries lyophilisées et on prépare les sphères comme décrit dans l'exemple 1. Rincées à l'eau distillée stérile après formation de la membrane par transacylation et neutralisation, les sphères de bactéries sont immédiatement utilisées pour les essais de fermentation du lait.

### * Essais de fermentation du lait

On utilise du lait demi-écrémé Nactalia, en briques stériles de 200 ml.

### Conduite de l'expérience (dupliquée)

On mesure le pH de divers échantillons de 50 ml de lait au temps zéro, après 24 h et après 30 h d'agitation magnétique douce à température du laboratoire:
- échantillons de lait pur (sans addition de microsphères, ni de bactéries)
- échantillons de lait additionnés d'un lot de microsphères contenant 240 mg de bactéries lactiques
- échantillons de lait additionnés de 240 mg de bactéries lactiques libres
- échantillons de lait additionnés d'un lot de microsphères sans bactéries.

### Résultats

Le tableau 1 rassemble les résultats moyens obtenus. Le pH initial du lait était de 6,75. On note qu'il évolue spontanément pour s'abaisser à 6 après 30 h (échantillons témoins: lait seul).

Quand on ajoute au lait des sphères sans bactéries, le pH s'abaisse légèrement moins que dans les échantillons témoins.

En revanche, on constate que les bactéries encapsulées ont une nette activité sur le pH du lait, plus bas après 30 h que dans le cas des échantillons additionnés de bactéries libres.

Il est possible que le contenu des sphères, lequel se liquéfie au cours de l'expérience, exerce un effet favorable en constituant un aliment pour les bactéries.

### Exemple 22 selon l'invention

Fabrication de particules à membrane formée à partir d'ovalbumine et de PGA et contenant des oeufs d' Artemia salina.

Cette expérience a été effectuée en utilisant des oeufs secs d'Artemia salina, commercialisés comme nourriture de poissons d'aquariums (Hobby).

### * Encapsulation

La solution de polymères est préparée comme décrit à l'exemple 20, mais en dissolvant du NaCl à la concentration de 3% dans les 12 ml d'eau, avant d'y dissoudre les polymères.

Dans la solution ainsi obtenue, on disperse 240 mg d'oeufs secs d'Artemia salina et on applique à cette suspension le protocole décrit à l'exemple 2. Les capsules obtenues sont rincées ensuite à l'eau, puis à l'eau additionnée de 3% de NaCl. Elles sont finalement dispersées dans de l'eau additionnée de 3% de NaCl et laissées à température du laboratoire.

### *Résultats

Les particules sont examinées au microscope à intervalles de temps réguliers. Moins de 24 h après l'encapsulation, les oeufs éclosent à l'intérieur des microcapsules et les mouvements des crustacés sont très visibles à travers la membrane transparente. Le procédé d'encapsulation est donc bien compatible avec le maintien de la viabilité d'organismes vivants.

### Exemple 23 selon l'invention

Fabrication de particules à membrane formée à partir de phosphatase alcaline et de PGA.

Le protocole décrit à l'exemple 3 est reproduit en remplaçant la sétumalbumine humaine par la phosphatase alcaline (type I-S, SIGMA).

Après liquéfaction du gel, les sphères liquéfiées sont rincées plusieurs fois à l'eau distillée. Elles sont ensuite congelées et lyophilisées.

### *Dosage de l'activité enzymatique des sphères de phosphatase alcaline

- Principe:

Le dosage consiste à effectuer une détermination spectrophotométrique (lecture à 405 nm) de la quantité de paranitrophénol (pNP) libéré par hydrolyse du paranitrophénylphosphate (pNPP) à 37°C.
- Préparation des réactifs

On prépare les réactifs suivants:
- un tampon glycine 100 mM, pH 10,4 , contenant MgCl2 1 mM et ZnCl2 1mM.
- Une solution de Mg Cl2 1 mM
- Une solution de paranitrophénylphosphate 60 mM
- Une solution de NaOH 0,2 M

### *Protocole de dosage

On effectue parallèlement une détermination de l'activité de la phosphatase alcaline libre (non encapsulée: PAL) et de l'activité de la phosphatase alcaline immobilisée sous forme de sphères lyophilisées.

Les conditions figurent dans le tableau 2.

Les quantités de pNP libérés dans les deux séries d'essais sont évaluées après 1 min, 2 min et 3 min. Tous les essais sont dupliqués.

### * Résultats

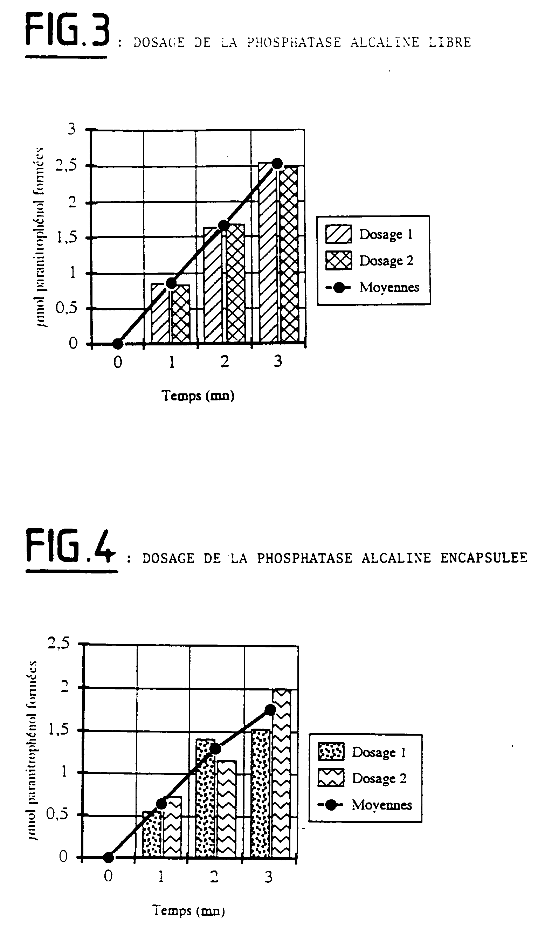

Ils sont regroupés sur le tableau 3 et les figures 3 et 4.

Les résultats sont exprimés en unités par mg d'enzyme pure ou de sphères, l'unité étant définie comme la quantité capable d'hydrolyser une µmole de substrat par minute dans les conditions expérimentales définies.

On observe donc que les sphères, avec une activité moyenne de 0,6227 unités par mg, ont une activité enzymatique égale à 11,1 % de celle de l'enzyme pure. Etant donné que 1 mg de sphères contient au maximum 0,625 mg de phophatase alcaline, l'enzyme encapsulée a conservé au minimum 17,7 % de son activité.

### Exemple 24 selon l'invention

Fabrication de particules à membrane formée à partir de PGA ajouté à la solution initiale, et d'ovalbumine ajoutée en phase externe.
a) Préparation de la solution initiale
   Dans 12 ml d'eau distillée on dissout:
   - de l'alginate de sodium à la concentration de 1%
   - du PGA à la concentration de 2 %.
b) Individualisation des sphères par traitement au chlorure de calcium
   On procède comme décrit à l'exemple 1.
c) Formation de la membrane
   Les sphères sont mises en suspension dans 50 ml d'une solution aqueuse d'ovalbumine à 5 %, pendant 5 min sous agitation magnétique. Puis
   - on ajoute 1,6 ml de NaOH 1 M, et on laisse agiter 15 min.
   - on ajoute 1,2 ml de HCl 1 M, et on laisse agiter 15 min.

   Les sphères à membrane obtenues sont rincées plusieurs fois à l'eau distillée.
d) Liquéfaction du gel
   On applique aux sphères le traitement au citrate de sodium décrit à l'exemple 2.

Après traitement au citrate, les sphères perdent leur aspect opalescent pour devenir transparentes. Elles se présentent alors comme des vésicules non sphériques dont la membrane est parfaitement visible. Après 5 min en suspension dans l'eau, les sphères reprennent une forme sphérique en gonflant dans l'eau. Leur diamètre devient environ le double de celui des sphères d'alginate-PGA obtenues dans la solution calcique. Ces sphères transparentes se colorent en bleu au contact d'une solution diluée de bleu de méthylène.

### Exemple 25 selon l'invention

Fabrication de particules à membrane formée à partir d'ovalbumine et de PGA et contenant des graines de Cresson.

Le protocole décrit à l'exemple 10 est reproduit en dispersant dans la phase aqueuse initiale 480 mg de graines de Cresson (Vilmorin).

Après individualisation dans la solution calcique, les sphères, blanches, contiennent un nombre variable de graines.

Après le traitement de liquéfaction au citrate, les sphères sont ramollies mais ne laissent pas échapper les graines. Après rinçage à l'eau et mise en suspension dans l'eau, les sphères regonflent instantanément.

Mise en germination: Dans trois boîtes de Pétri garnies de coton hydrophile imbibé d'eau, on place respectivement, à température ambiante (20°C):
- une dizaine de sphères chargées de graines et non traitées au citrate,
- une dizaine de sphères chargées de graines et traitées au citrate,
- et des graines non encapsulées.

Après 24 h, tandis que les graines non encapsulées ne sont pas modifiées, dans les sphères, traitées ou non par le citrate, on aperçoit par transparence au travers des membranes, des germes sortant des graines. Après 3 jours, les graines nues ont commencé à germer tandis que les germes des graines encapsulées ont percé la membrane et sont déjà bien développés.

### Exemple 26 selon l'invention

Fabrication de particules à membrane formée à partir d'ovalbumine et de PGA et contenant des fibroblastes de peau humains.

L'ensemble des opérations décrites ci-dessous est réalisé dans des conditions stériles: manipulations sous une hotte à flux laminaire, utilisation de matériel préalablement stérilisé.
a) Préparation de la phase aqueuse initiale
   Dans 12 ml de PBS ("phosphate buffered saline", sans calcium ni magnésium, Gibco), on dissout:
   - 240 mg de PGA
   - 120 mg d'alginate de sodium (Laserson et Sabetay)
   - 720 mg d'albumine de blanc d'oeuf (Laserson et Sabetay).

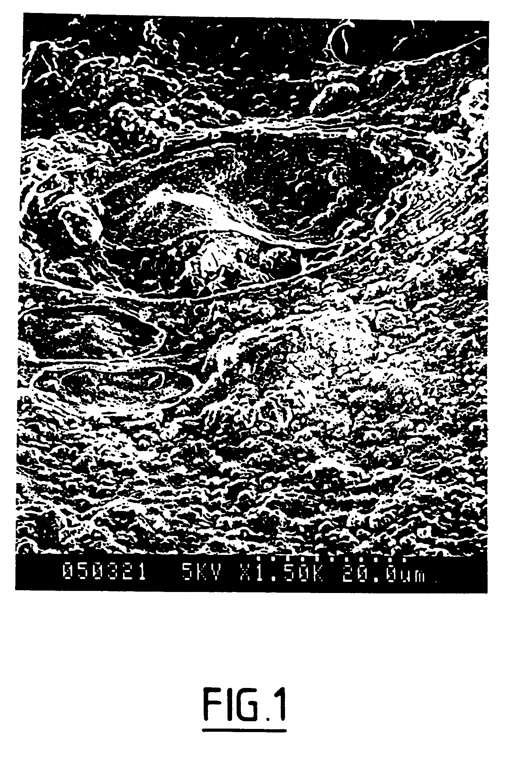

   La solution est centrifugée pendant quelques minutes à faible vitesse, de façon à éliminer les bulles d'air.
b) Incorporation des fibroblastes
   Des fibroblastes de peau humains, préalablement cultivés en monocouches dans des boîtes de culture, sont recueillis par un traitement à la trypsine, puis mis en suspension dans du sérum de veau foetal, et dispersés dans la solution précédente à raison de 50.000 cellules/ ml.
c) Formation des sphères
   Cette suspension est versée goutte à goutte à l'aide d'une seringue et d'une aiguille dans 50 ml d'une solution aqueuse de CaCl2 à 10%, agitée par agitation magnétique. L'agitation est poursuivie pendant 5 min. Les sphères sont ensuite rincées plusieurs fois à l'eau distillée.
d) Formation de la membrane
   Les sphères sont remises en suspension dans 50 ml d'eau distillée avec agitation magnétique. On ajoute à la suspension 50 µl de NaOH 1M et on laisse la réaction se développer pendant 5 min. On ajoute alors à la suspension 50 µl de HCl 1M et on laisse l'agitation se poursuivre pendant 5 min. Les sphères sont ensuite rincées plusieurs fois à l'eau distillée puis au PBS. Après incision de la membrane, on voit bien les cellules encapsulées, qui apparaissent comme de petites sphères réfringentes.
e) Mise en culture
   Les sphères contenant les cellules sont mises à incuber dans un milieu de culture (DMEM, Gibco) contenant 10 % de sérum de veau foetal et 2 mM de glutamine. L'ensemble est introduit dans un incubateur, en atmosphère contenant 5 % de CO2, à 37°C. Le milieu de culture est changé régulièrement. L'examen périodique des sphères à chaque changement de milieu montre une augmentation progressive du nombre de cellules encapsulées. Les fibroblastes prolifèrent donc régulièrement à l'intérieur des sphères, comme le montre le cliché de la figure 1, obtenu en microscopie électronique à balayage.

### Exemple 27 selon l'invention

Fabrication de particules de diamètre 20 -100 µm, à membrane formée à partir d'ovalbumine et de PGA.
a) Préparation de la solution aqueuse initiale
   Dans 50 ml d'eau distillée, on dissout:
   - 500 mg de PGA
   - 500 mg d'alginate de sodium
   - 2,5 g d'ovalbumine

   On agite jusqu'à dissolution, puis on laisse les bulles d'air s'éliminer spontanément pendant 15 min.
b) Formation des microsphères
   On pulvérise cette solution en fines gouttelettes, à l'aide d'un pulvérisateur à air comprimé, dans 500 ml d'une solution aqueuse de CaCl2 à 10 %, sous agitation. On maintient l'agitation pendant 10 min.
c) Formation de la membrane
   On ajoute 8 ml de NaOH 1M, et on agite 15 min.
   On ajoute 8 ml de HCl 1M, et on agite 15 min.
   Les microsphères à membrane sont ensuite centrifugées et rincées plusieurs fois à l'eau distillée.
d) liquéfaction du gel
   La moitié du sédiment est remise en suspension dans 50 ml d'une solution aqueuse de citrate de sodium à 10 % pendant 10 min, puis les microsphères liquéfiées sont rincées plusieurs fois à l'eau distillée et conservées en suspension aqueuse. On obtient des microsphères à membranes dont le diamètre est compris entre 20 et 100 µm.

### Exemple 28 selon l'invention

Fabrication de particules à membrane formée à partir d'ovalbumine et de PGA.

Le protocole décrit à l'exemple 10 est appliqué, en supprimant l'alginate de sodium de la phase aqueuse initiale et en utilisant le PGA à la concentration de 5 % et l'ovalbumine à la concentration de 5 % également. L'étape de gélification est poursuivie pendant 30 min en augmentant à 30 % la concentration de la solution de chlorure de calcium. On obtient des sphères stables à membrane bien visible.

### Exemple 29 selon l'invention

Fabrication de particules à membrane formée à partir d'ovalbumine et contenant de l'huile de bourrache.
a) Préparation de la solution aqueuse initiale
   Dans 600 ml d'eau distillée, on dissout:
   - 12 g de PGA (Kelcoloïd S, Kelco)
   - 6 g d'alginate de sodium (Manucol DH, Kelco)
   - 36 g d'ovalbumine (Laserson et Sabetay)

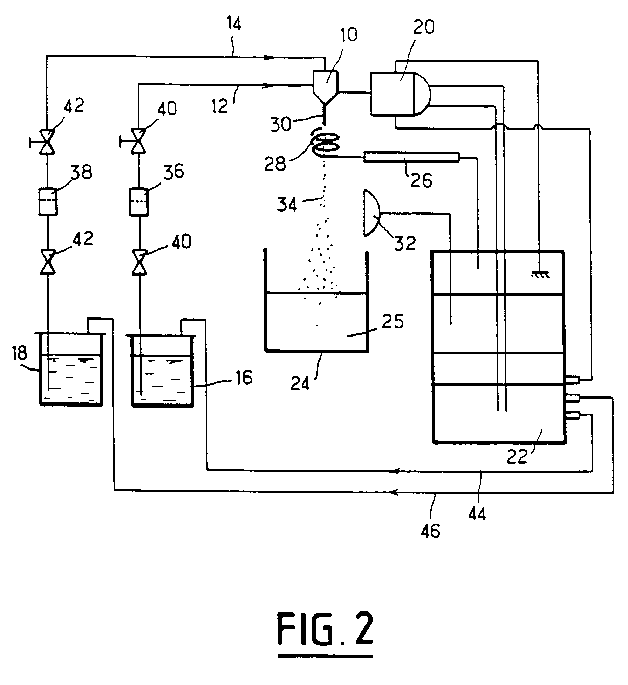

   On agite jusqu'à dissolution, puis on laisse les bulles d'air s'éliminer.
b) Formation des sphères par coextrusion et gélification
   Pour ce faire, on utilise l'appareil Extramet schématisé à la figure 2 annexée. L'homme de l'art pourra également se reporter au document WO 92/06771 qui comprend une figure identique. Cet appareil comprend essentiellement une buse d'extrusion 10 permettant de réaliser une coextrusion par la présence de deux orifices concentriques alimentés séparément par deux conduits d'alimentation 12 et 14 servant respectivement à l'alimentation externe en solution ovalbumine-PGA-alginate de sodium selon l'invention depuis un réservoir 16, et à l'intérieur, de l'huile de bourrache depuis un réservoir 18. A cette buse 10, est associé un dispositif vibreur 20 commandé par des moyens de commande 22. Ce dispositif comprend également un bain gélifiant 24 disposé à distance sous la buse 10, dans lequel est placée la solution 25 de CaCl2 à 10 %. Cet appareil comprend également une électrode 26 à extrémité hélicoïdale 28 disposée concentriquement à l'écoulement du flux laminaire 30 coextrudé de la base 10 de manière à maintenir séparées les gouttelettes générées par le vibreur 20. On peut également prévoir un dispositif de stroboscopie par flash 32 pour observer visuellement les gouttelettes 34 ainsi générées tombant dans le bain de gélification. Le débit de la solution ovalbumine-PGA-alginate de sodium présente dans le réservoir 16 est de 11/h et celui de l'huile de bourrache présente dans le réservoir 18 est de 250 ml/h. La fréquence de vibration du vibreur 20 est 230 Hz.Les diamètres des deux orifices concentriques sont de 350 et 400 µm.
   Les gouttelettes 34 générées par le vibreur 20 à partir de l'écoulement laminaire réalisé dans la buse de coextrusion 10 sont reçues dans 1l de solution de gélification 25 maintenue sous agitation magnétique. Le bain est renouvelé après extrusion de 250 g de la solution ovalbumine-PGA-alginate de sodium. Les sphères formées sont lavées plusieurs fois à l'eau distillée.
c) Formation de la membrane
   Les sphères sont mises en suspension dans 2,5 l d'eau distillée sous agitation magnétique.
   - On ajoute 20 ml de NaOH 1 N, et on laisse agiter 15 min.
   - On ajoute 7,5 ml de HCl 1 N, et on laisse agiter 15 min.

   Les sphères obtenues sont rincées plusieurs fois à l'eau distillée. On obtient des sphères lisses, de diamètre 1,5 mm, renfermant une gouttelette d'huile de bourrache.

### Exemple 30 selon l'invention

Fabrication de particules à membrane formée à partir d'ovalbumine
a) Préparation de la solution aqueuse initiale
   Dans 600 ml d'eau distillée, on dissout:
   - 12 g de PGA (Kelcoloïd S, Kelco)
   - 6 g d'alginate de sodium (Manucol DH, Kelco)
   - 36 g d'ovalbumine (Laserson et Sabetay)

   On agite jusqu'àdissolution, puis on laisse les bulles d'air s'éliminer.
b) Formation des sphères par extrusion et gélification
   On réalise l'extrusion et la gélification des sphères dans les mêmes conditions que celles décrites à l'exemple 29, sauf en ce qui concerne la buse d'extrusion 10 qui ne comporte qu'un seul orifice de 250 µm.
c) Formation de la membrane
   Le protocole de formation de la membrane décrit à l'exemple 29 est appliqué. On obtient des sphères lisses, opalescentes, de diamètre 1,4 mm.

### Exemple 31 selon l'invention

Fabrication de particules à membrane formée à partir de chitosan.

On prépare une solution de chitosan (Seacure 143, Pronova Biopolymer) à 5 % dans l'acide acétique 1 M. La solution est ajustée jusqu'à pH 5,6 à l'aide de soude 5 M, puis additionnée de 2 % de PGA. 12 ml de cette solution sont ajoutés goutte à goutte à 50 ml de soude 1 M, sous agitation magnétique. L'agitation est maintenue pendant 10 min. Puis on neutralise la suspension à l'aide d'HCl 1 M. Les billes obtenues sont rincées plusieurs fois à l'eau. Les billes sont blanches opaques. On met en évidence la présence d'une membrane après trempage des billes dans une solution d'HCl de pH 2: après 5 min, le chitosan emprisonné à l'intérieur de la membrane s'est dissous et on obtient des vésicules transparentes à membrane formée de PGA associé au chitosan.

### Exemple 32 selon l'invention

Fabrication de particules à membrane formée à partir de polyéthylène imine.

On prépare une solution à 2 % de PGA et 1 % d'alginate de sodium dans l'eau distillée. 12 ml de cette solution sont ajoutés goutte à goutte à 50 ml de soude 1 M. Après 10 min d'agitation magnétique, les billes obtenues sont rincées à l'eau. Les billes sont remises en suspension dans un bain alcalin constitué de 50 ml d'eau additionnés de 500 mg d'une solution commerciale (SIGMA) de polyéthylène imine à 50 % (p/v) dans l'eau distillée. Une agitation magnétique est installée. Après 20 min, la suspension est neutralisée au moyen d'HCl 1 M. On obtient des billes translucides à fine membrane. Après liquéfaction au moyen de citrate, on obtient des vésicules complètement transparentes et de diamètre augmenté.

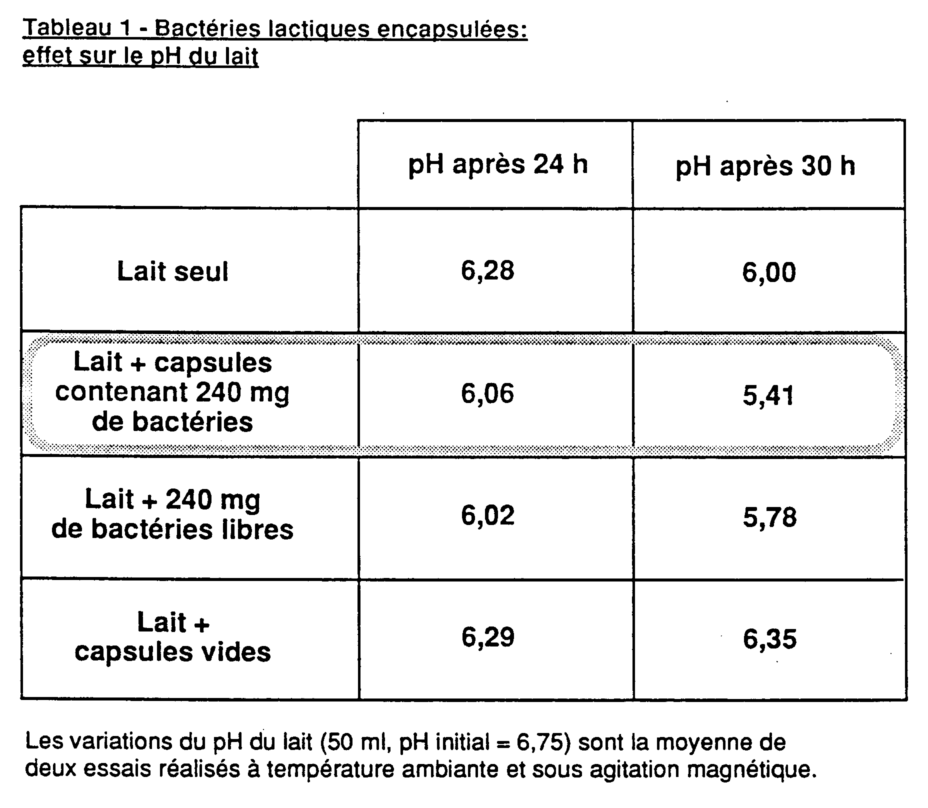

**Tableau 2**

| Protocole de dosage de l'activité enzymatique des sphères de phosphatase alcaline | | | | |
|---|---|---|---|---|
| | **Dosage PAL******* | **Blanc PAL** | **Dosage BILLES** | **Blanc BILLES** |
| Tampon Glycine pH 10.4 | 2.6 ml | 2.6 ml | 13 ml | 13 ml |
| PAL dans MgCl₂ 1mM | 100µl | --- | --- | --- |
| Billes PAL | --- | --- | 15 mg | --- |
| MgCl₂ 1mM | --- | 100 µl | 500 µl | 500 µl |
| | Mise à température 15 mn au bain-marie à 37°C | | | |
| Solution de pNPP 60mM | 300 µl | 300 µl | 1.5 ml | 1.5 ml |
| *Nombre de µmol de pNPP* | *18 µmol* | *18 µmol* | *90 µmol* | *90 µmol* |
| *Concentration pNPP finale* | *6mM* | *6mM* | *6mM* | *6mM* |
| Arrêt: NaOH 0.2N | 3 ml après 1 ou 2 ou 3 mn | | 15 ml après 1 ou 2 ou 3 mn | |
| Dilution | 1/20e ou 1/40e avec NaOH 0.2 N dans fioles jaugées de 20 ml | | | |
| Dosage | Lecture D.O. au spectrophotomètre à 405 nm contre le blanc | | | |

| | | | | |
|---|---|---|---|---|
| *PAL = phosphatase alcaline libre (non encapsulée) | | | | |

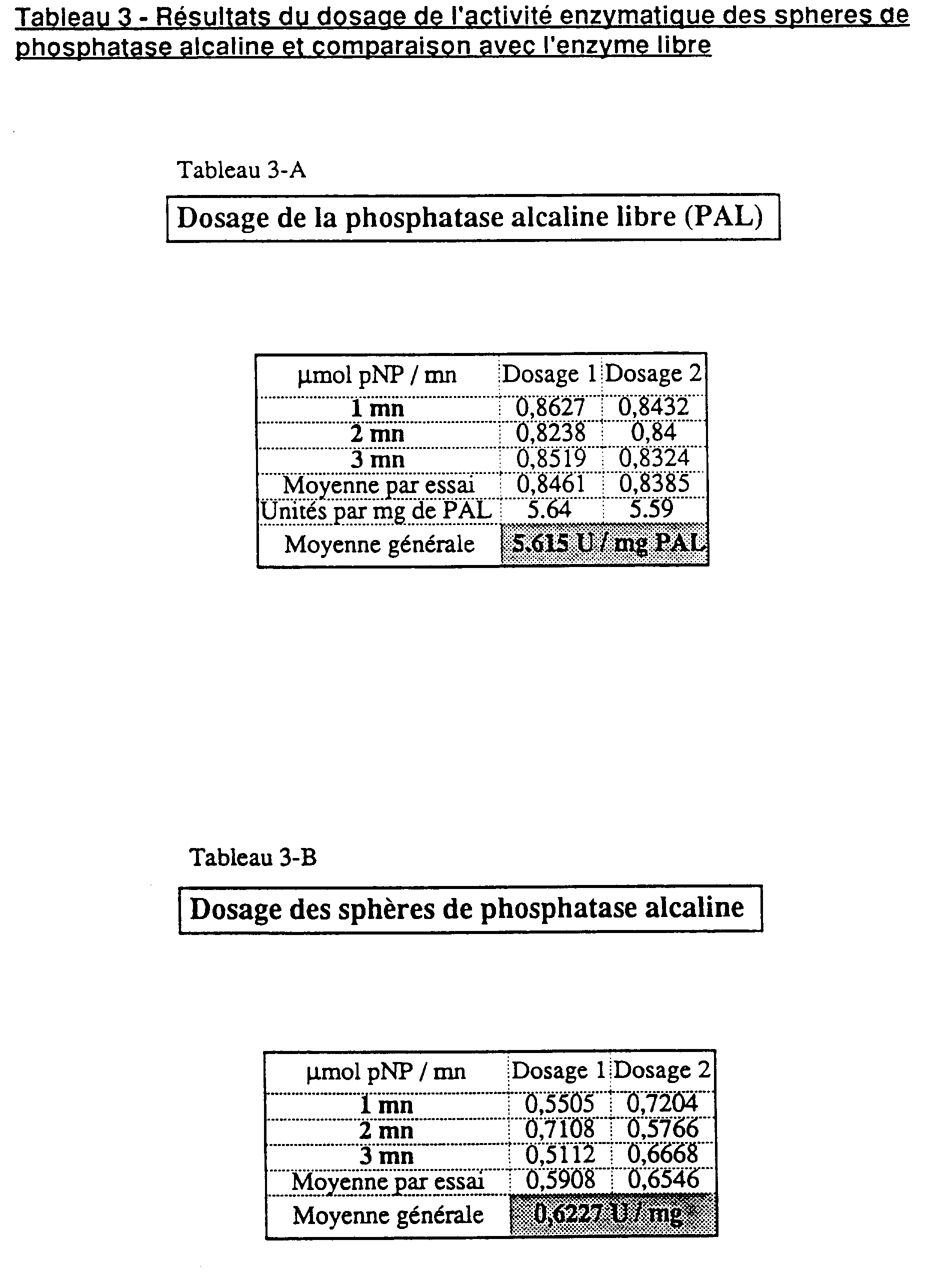

## Revendications

1. Particule, notamment bille, capsule, microcapsule, sphère ou microsphère, caractérisée en ce qu'elle comprend au moins un polysaccharide estérifié et au moins une polyamine, additionnés d'au moins un polysaccharide gélifiable lorsque ni le polysaccharide estérifié ni la polyamine ne sont gélifiables dans les conditions opératoires choisies, la dite particule comprenant au moins en surface une membrane formée par le produit de la réaction de transacylation avec formation de liaisons covalentes amide entre le polysaccharide estérifié et la dite polyamine au sein d'un gel éventuellement liquéfiable.

2. Particule selon la revendication 1, caractérisée en ce que le polysaccharide estérifié précité est un polysaccharide hydrophile porteur de groupements carboxyliques qui sont estérifiés dans une proportion au moins égale à 50%.

3. Particule selon l'une des revendications 1 et 2, caractérisée en ce que le polysaccharide estérifié précité peut être choisi parmi le groupe consistant d'un alginate estérifié, en particulier d'un alginate de propylène glycol, une pectine, en particulier une pectine fortement méthoxylée, ou tout autre composé obtenu par estérification de carboxyles de polysaccharides porteurs de carboxyles.

4. Particule selon l'une des revendications 1 à 3, caractérisée en ce que la polyamine précitée comprend une protéine, un polypeptide, un polyaminoacide, un polysaccharide porteur de groupements aminés tel que le chitosan, une substance organique, aliphatique, alicyclique ou aromatique porteuse de plusieurs groupements aminés primaires ou secondaires, telle que l'éthylènediamine, la pentaméthylènediamine, l'hexaméthylènediamine, la pipérazine, la phénylènediamine, la polyéthylèneimine.

5. Particule selon la revendications 4, caractérisée en ce que la protéine précitée est choisie parmi une protéine hydrophile ou traitée de manière à être rendue hydrophile, c'est-à-dire soluble dans l'eau ou dispersible dans l'eau, contenant des groupements aminés libres, de préférence choisie parmi le groupe consistant d'albumines comme la sérumalbumine, l'ovalbumine, l'alpha-lactalbumine, des globulines, le fibrinogène, la caséine, des scléroprotéines solubilisées, le collagène, l'atélocollagène, la gélatine, les hydrolysats de gélatine, les peptones, l'hémoglobine, des protéines végétales telles que les protéines du soja ou du blé, des gluténines, qui de préférence auront été dégradées, des enzymes telles que la catalase, les phosphatases, des hormones, des immunoglobulines ou des anticorps tels que des anticorps monoclonaux, des polypeptides, des polyaminoacides, ou des mélanges naturels ou non renfermant une ou plusieurs protéines tels que les mélanges d'atélocollagène et de glycosaminoglycannes, le lait entier ou écrémé totalement ou partiellement, le lait condensé, le lait en poudre, les protéines du lactosérum, l'oeuf entier, le blanc d'oeuf, le jaune d'oeuf, la farine de soja, les concentrés protéiques de soja, les préparations alimentaires liquides au soja, le lait de coco, du sérum sanguin, du bouillon de viande, des milieux de culture, en particulier des milieux pour culture de cellules ou de tissus végétaux, de cellules ou de tissus animaux ou des milieux pour culture de microorganismes.

6. Particule selon l'une des revendications 1 à 5, caractérisée en ce que le polysaccharide précité est un polysaccharide gélifiable, en particulier choisi parmi le groupe consistant d'un alginate, d'un carraghénane, en particulier le kappa-carraghénane, une pectine gélifiable, en particulier une pectine faiblement méthoxylée, un polysaccharide porteur de groupements aminés de préférence gélifiable pour un pH supérieur à 6,2 ou en présence d'un polyphosphate, tel que le chitosan.

7. Particule selon l'une des revendications 1 à 6, caractérisée en ce que la membrane précitée emprisonne un contenu liquide, constitué d'un gel liquéfié ou d'un liquide encapsulé aqueux ou de nature hydrophobe.

8. Particule selon l'une des revendications 1 à 6, caractérisée en ce que la membrane précitée enveloppe un noyau gélifié comprenant soit le polysaccharide gélifiable précité, soit le polysaccharide estérifié précité, soit encore la polyamine précitée, sous une forme gélifiée par une entité de gélification.

9. Particule selon l'une des revendications 1 à 6, caractérisée en ce qu'elle est constituée d'un gel rigidifié dans toute sa masse, composé du produit de la réaction dite de transacylation entre le polysaccharide estérifié et la polyamine éventuellement additionnés d'un polysaccharide gélifiable.

10. Particule selon l'une des revendications précédentes, caractérisée en ce que la proportion relative en poids de polysaccharide estérifié par rapport à la polyamine est comprise entre 3 % et 500 % en poids.

11. Particule selon l'une des revendications précédentes, caractérisée en ce que la proportion relative en poids de polysaccharide gélifiable par rapport au polysaccharide estérifié est comprise entre 0 % et 300 % en poids.

12. Particule selon l'une des revendications précédentes, caractérisée en ce qu'elle contient un ou plusieurs principe(s) actif(s), à l'état de solution, de suspension ou d'émulsion, en particulier choisi parmi le groupe consistant d'un principe actif cosmétique, pharmaceutique, une substance d'intérêt agroalimentaire, une substance intéressant le diagnostic ou les réactifs, une protéine ayant une activité biologique telle qu'une enzyme, une hormone, un anticorps ou l'hémoglobine, des particules insolubles, par exemple des particules d'un matériau adsorbant tel que le charbon actif, une phase liquide hydrophobe, telle qu'une huile végétale, une huile minérale, une huile de silicone, une huile essentielle, ou une solution d'une ou plusieurs substances dans une phase liquide hydrophobe, une mousse contenant des bulles de gaz tel que l'air, un vaccin pour relargage lent in situ, ou des matériels vivants tels que des microorganismes, des cellules, tissus, organes appartenant au règne animal ou végétal, ou des constituants cellulaires tels que les microsomes hépatiques pour effectuer des bioconversions, ou du matériel génétique appartenant au règne animal ou au règne végétal.

13. Particule selon la revendication 12, caractérisée en ce que les matériels vivants précités comprennent des microorganismes pour réaliser des synthèses ou des bioconversions, tels que des bactéries comme celles utilisées pour les fermentations de produits laitiers, ou celles utilisées pour la dépollution des eaux, ou des champignons tels que les mycorhizes ou les levures comme par exemple les levures utilisées dans la fabrication de la bière, ou les levures utilisées pour la prise de mousse du champagne, ou des micro-algues, des graines éventuellement additionnées de substances diverses protectrices ou influençant leur germination ou leur croissance, des embryons somatiques végétaux pour produire des semences synthétiques, des apex végétaux, des cellules ou des tissus végétaux notamment pour effectuer des biosynthèses ou des bioconversions, du matériel vivant appartenant au règne animal, tel que des cellules ou des tissus utilisables notamment pour effectuer des tests de toxicologie in vitro comme les cellules hépatiques, les chondrocytes, les neurones, des cellules ou des groupes de cellules ou des tissus utilisables dans le cadre d'une thérapie cellulaire comme les îlots de Langerhans pour le traitement du diabète, les cellules de la médullosurrénale ou les cellules chromaffines pour le traitement de la maladie de Parkinson ou pour le traitement des douleurs chroniques, ou des cellules, génétiquement modifiées ou non, utilisables pour la production de substances biologiques telles que des hormones, des enzymes, des facteurs de croissance, de l'interféron, des facteurs de coagulation, ou des hybridomes pour la production d'anticorps monoclonaux ou des oeufs, des gamètes, ou des embryons.

14. Particule selon l'une quelconque des revendications 1 à 13, caractérisée en ce qu'elle présente une partie centrale constituée d'une phase aqueuse éventuellement gélifiée ou liquéfiée, contenant un matériel en particulier un matériel vivant tel que des microorganismes comme des levures ou des bactéries, ou des cellules vivantes, en particulier des cellules ou tissus végétaux, des cellules ou tissus animaux, ou des groupes de cellules ou tissus, ou une substance biologique comme par exemple une enzyme, et une couche externe ne contenant pas ledit matériel et comportant au moins en surface une membrane formée par le produit de la réaction de transacylation avec formation de liaisons covalentes amide entre au moins un polysaccharide estérifié et au moins une polyamine.

15. Procédé de fabrication de particules, en particulier de billes, capsules, microcapsules, sphères ou microsphères, caractérisé en ce qu'il comprend les étapes successives suivantes:
a) on prépare une première solution aqueuse initiale gélifiable par un agent de gélification, contenant au moins un polysaccharide estérifié et au moins une polyamine, additionnés d'au moins un polysaccharide gélifiable lorsque ni le polysaccharide estérifié ni la polyamine ne sont gélifiables dans les conditions opératoires choisies,
b) on fait tomber des gouttes de cette première solution dans une deuxième solution aqueuse formant bain de gélification contenant un agent de gélification, de manière à individualiser des particules par gélification à l'aide dudit agent de gélification,
c) on met en contact les particules gélifiées obtenues avec une solution aqueuse alcaline, de manière à déclencher une réaction dite de transacylation, au moins à la surface des particules gélifiées, entre le polysaccharide estérifié et la polyamine contenus dans lesdites particules, pendant une période de temps prédéterminée, pour former une membrane au moins en surface desdites particules,
d) on ajoute au milieu réactionnel un agent acide de manière à neutraliser et ainsi stabiliser les particules, en obtenant ainsi des particules comprenant au moins en surface une membrane formée par le produit de réaction avec formation de liaisons covalentes entre le polysaccharide estérifié et la polyamine.

16. Procédé selon la revendication 15, caractérisé en ce que les gouttes de solution gélifiable sont formées par tout moyen d'individualiser les gouttes tels que les systèmes d'injection comprenant une seringue munie d'une aiguille ou une pompe péristaltique équipée d'un tube muni d'une aiguille ou un système de dispersion par utilisation d'un pulvérisateur à air comprimé ou encore en coupant mécaniquement à l'aide d'un vibreur un écoulement laminaire réalisé soit par extrusion d'une solution à travers une buse.

17. Procédé selon la revendication 15 pour la fabrication de particules, en particulier de billes, capsules, microcapsules, sphères ou microsphères, contenant une phase liquide encapsulée, aqueuse ou hydrophobe, caractérisé en ce qu'il comprend les étapes successives suivantes:
a) on prépare une première solution aqueuse initiale gélifiable par un agent de gélification, contenant au moins un polysaccharide estérifié et au moins une polyamine, additionnés d'au moins un polysaccharide gélifiable lorsque ni le polysaccharide estérifié ni la polyamine ne sont gélifiables dans les conditions opératoires choisies ;
b) on prépare une phase liquide, aqueuse ou hydrophobe à encapsuler ;
c) on réalise à travers une buse d'extrusion une coextrusion laminaire de ladite première solution aqueuse en flux externe, initiale et de la phase liquide aqueuse ou hydrophobe à encapsuler en flux interne, dans des conditions réalisant une dissociation d'écoulement laminaire en gouttes individuelles, par exemple en soumettant l'écoulement laminaire à des vibrations.
d) on fait tomber ces gouttes dans une deuxième solution aqueuse formant bain de gélification contenant un agent de gélification, de manière à individualiser des particules par gélification à l'aide dudit agent de gélification, lesdites particules contenant ladite phase liquide à encapsuler.
e) on met en contact les particules gélifiées obtenues avec une solution aqueuse alcaline, de manière à déclencher une réaction dite de transacylation, au moins à la surface des particules gélifiées, entre le polysaccharide estérifié et la polyamine contenus dans lesdites particules, pendant une période de temps prédéterminée, pour former une membrane au moins en surface desdites particules,
f) on ajoute au milieu réactionnel un agent acide de manière à neutraliser et ainsi stabiliser les particules, en obtenant ainsi des particules comprenant au moins en surface une membrane formée par le produit de réaction avec formation de liaisons covalentes entre le polysaccharide estérifié et la polyamine et renfermant une goutte du liquide à encapsuler précité.

18. Procédé selon la revendication 17 pour la fabrication de particules, en particulier de billes, capsules, microcapsules, sphères ou microsphères, contenant un matériel à encapsuler présent exclusivement dans sa partie centrale, caractérisé en ce que dans l'étape b) on prépare une phase aqueuse à encapsuler renfermant éventuellement un polysaccharide gélifiable et contenant un matériel, en particulier un matériel vivant tel que des microorganismes comme des levures ou des bactéries, ou des cellules ou des tissus vivants, animaux ou végétaux, ou une substance biologique comme par exemple une enzyme.

19. Procédé selon l'une des revendications 15 à 18, caractérisé en ce qu'on ajoute une polyamine à la solution aqueuse alcalisée qui est mise en contact avec les particules gélifiées.

20. Procédé selon l'une des revendications 15 à 19, caractérisé en ce que l'agent de gélification est un cation mono ou polyvalent, en particulier choisi parmi le groupe consistant d'un sel de calcium ou d'un sel de potassium, ou d'un sel de fer, ou d'aluminium, ou de cuivre, ou de manganèse ou de baryum, ou une solution aqueuse de pH supérieur à 6,2 ou une solution de polyphosphate, selon la nature du constituant gélifiant.

21. Procédé selon l'une des revendications 15 à 20, caractérisé en ce que la concentration en agent de gélification en particulier en cation mono ou polyvalent ou en polyphosphate dans la solution aqueuse formant bain gélifiant précité, est comprise entre 0,01 M et 3M, de préférence entre 0,8 M et 1 M.

22. Procédé selon l'une des revendications 15 à 21, caractérisé en ce que le temps pendant lequel les particules sont agitées dans le bain gélifiant est compris entre 1 et 40 minutes.

23. Procédé selon l'une des revendications 15 à 22, caractérisé en ce que la concentration en polysaccharide gélifiable précité dans la solution aqueuse initiale à disperser et gélifier est comprise entre 0 et 20 %, de préférence entre 0,5% et 2%, encore mieux d'environ 1 % en poids/v.

24. Procédé selon l'une des revendications 15 à 23, caractérisé en ce que la concentration en polysaccharide estérifié dans la solution aqueuse initiale précitée est comprise entre 0,5 % et 20 % en poids et encore de préférence est voisine de 2 % en poids/v.

25. Procédé selon l'une des revendications 15 à 24, caractérisé en ce que la concentration en polyamine dans la solution aqueuse initiale précitée ou dans la solution dans laquelle sont dispersées les particules gélifiées, est comprise entre 1 % et 30 % en poids/v.

26. Procédé selon l'une des revendications 15 à 25, caractérisé en ce que la solution alcaline déclenchant la réaction précitée de transacylation avec formation de liaisons covalentes au moins à la surface des particules présente un pH compris entre 8 et 14, encore de préférence entre 10,5 et 12,5.

27. Procédé selon la revendication 26, caractérisé en ce que la solution aqueuse alcaline est formée par ajout d'un agent alcalin pour amener le pH à la valeur de pH précitée, cette solution aqueuse alcaline pouvant être éventuellement obtenue par ajout de l'agent alcalin dans la solution aqueuse formant bain gélifiant précité.

28. Procédé selon la revendication 27, caractérisé en ce que l'agent alcalin peut être choisi par exemple parmi la soude, la potasse, l'ammoniaque, ou un composé organique aminé tel que par exemple la triéthanolamine, la triéthylamine, la polyéthylèneimine.

29. Procédé selon l'une des revendications 15 à 28, caractérisé en ce que le temps pendant lequel les particules sont maintenues au sein de la solution alcaline pour que se développe la réaction de transacylation est compris entre 5 min et 1 heure, de préférence entre 5 min et 30 min, de préférence encore, il est de 15 min.

30. Procédé selon l'une des revendications 15 à 29, caractérisé en ce que l'épaisseur de la membrane, sa résistance à la lyse enzymatique et l'étroitesse de ses pores peuvent être augmentées par augmentation de la durée de ladite réaction de transacylation et/ou par des variations de la composition de la solution alcaline déclenchant ladite réaction de transacylation, et en particulier, par augmentation de la quantité d'agent alcalin utilisé pour préparer la dite solution alcaline.

31. Procédé selon l'une des revendications 15 à 30, caractérisé en ce que la quantité d'agent acide ajoutée à la suspension alcaline de particules après la réaction précitée de transacylation avec formation de liaisons covalentes est telle que l'eau de suspension des particules soit neutralisée ou amenée à un pH légèrement acide.

32. Procédé selon l'une des revendications 15 à 31, caractérisé en ce que l'agent acide précité utilisé pour neutraliser la suspension alcaline de particules après la réaction dite de transacylation avec formation de liaisons covalentes, est par exemple choisi parmi un acide organique monocarboxylique ou polycarboxylique, porteur ou non de fonction alcool, et en particulier choisi parmi le groupe consistant de l'acide acétique, acide citrique, acide tartrique, acide succinique, acide malique, acide lactique, ou un acide minéral comme l'acide chlorhydrique ou l'acide sulfurique.

33. Procédé selon l'une des revendications 15 à 32, caractérisé en ce que le temps de neutralisation des particules, c'est-à-dire le temps d'agitation nécessaire après ajout de l'acide au milieu réactionnel est compris entre 5 min et 1 heure, de préférence entre 5 min et 30 min, de préférence encore, il est de 15 min.

34. Procédé selon l'une des revendications 15 à 33, caractérisé en ce qu'il comprend l'étape supplémentaire de liquéfaction du contenu des particules, après formation de la membrane précitée par la réaction de transacylation avec formation de liaisons covalentes, avec un agent liquéfiant.

35. Procédé selon la revendication 34, caractérisé en ce que l'agent liquéfiant précité est une solution aqueuse de citrate ou de phosphate contenant en particulier une concentration de citrate ou de phosphate comprise entre 0,01 M et 1 M, et de préférence entre 0,2 M et 0,5 M.

36. Procédé selon l'une des revendications 15 à 35 caractérisé en ce que, dans la solution aqueuse initiale préparée à l'étape (a) précitée ou dans la phase liquide aqueuse ou hydrophobe à encapsuler par coextrusion, on introduit un ou plusieurs principes actifs directemeent ou à l'état de solution, de suspension ou d'émulsion, en particulier une ou plusieurs substances d'intérêt cosmétique, pharmaceutique, biomédical, ou agroalimentaire, des substances utilisables pour le diagnostic ou comme réactifs, des particules d'un matériau adsorbant tel que du charbon actif, une mousse contenant des bulles de gaz tel que l'air, un liquide hydrophobe, un vaccin, des matériels vivants tels que des microorganismes, des graines éventuellement additionnées de substances diverses protectrices ou influençant leur germination ou leur croissance, des cellules, tissus, ou organes du règne animal ou du règne végétal, des gamètes, des embryons, des constituants cellulaires.

37. Procédé selon l'une des revendications 15 à 36, caractérisé en ce que la polyamine précitée est une protéine douée d'une activité biologique spécifique telle qu'une enzyme, une hormone, un anticorps tel qu'un anticorps monoclonal, l'hémoglobine, seule ou mélangée à une autre protéine.

38. Procédé selon l'une des revendications 15 à 37, caractérisé en ce que les particules peuvent être séchées, par exemple par lyophilisation, nébulisation, ou dans un séchoir à lit fluidisé, par exemple à air avantageusement chauffé à une température appropriée.

39. Composition telle qu'une composition cosmétique, pharmaceutique, thérapeutique, alimentaire, enzymatique, composition pour thérapie cellulaire, composition pour thérapie enzymatique, composition pour épuration sanguine, composition pour réactifs, composition pour tests toxicologiques in vitro, compositions pour agriculture, compositions pour graines enrobées, composition pour production biotechnologique, caractérisée en ce qu'elle comprend des particules définies à l'une quelconque des revendications 1 à 14.

## Claims

1. Particle, especially a bead, capsule, microcapsule, sphere or microsphere, characterized in that it comprises at least one esterified polysaccharide and at least one polyamine, having added to them at least one polysaccharide which is gellable when neither the esterified polysaccharide nor the polyamine are gellable under the operating conditions chosen, the said particle comprising, at least at the surface, a membrane formed by the product of the transacylation reaction with formation of covalent amide bonds between the esterified polysaccharide and the said polyamine, within a gel which can possibly be liquefied.

2. Particle according to Claim 1, characterized in that the abovementioned esterified polysaccharide is a hydrophilic polysaccharide bearing carboxylic groups which are esterified to a proportion at least equal to 50%.

3. Particle according to one of Claims 1 and 2, characterized in that the abovementioned esterified polysaccharide may be chosen from the group consisting of an esterified alginate, in particular a propylene glycol alginate, a pectin, in particular a high methoxyl pectin, or any other compound obtained by esterifying carboxyls of carboxyl-bearing polysaccharides.

4. Particle according to one of Claims 1 to 3, characterized in that the abovementioned polyamine comprises a protein, a polypeptide, a polyamino acid, a polysaccharide bearing amino groups such as chitosan, or an aliphatic, alicyclic or aromatic organic substance bearing several primary or secondary amino groups, such as ethylenediamine, pentamethylenediamine, hexamethylenediamine, piperazine, phenylenediamine or polyethyleneimine.

5. Particle according to Claim 4, characterized in that the abovementioned protein is chosen from a hydrophilic protein or one treated so as to be rendered hydrophilic, that is to say water-soluble or dispersible in water, containing free amino groups, preferably chosen from the group consisting of albumins such as serum albumin, ovalbumin and alpha-lactalbumin, globulins, fibrinogen, casein, solubilized scleroproteins, collagen, atelocollagen, gelatin, gelatin hydrolysates, peptones, haemoglobin, plant proteins such as soya or wheat proteins, glutenins, which have preferably been degraded, enzymes such as catalase, phosphatases, hormones, immunoglobulins or antibodies such as monoclonal antibodies, polypeptides, polyamino acids, or natural or non-natural mixtures containing one or more proteins such as mixtures of atelocollagen and glycosaminoglycans, whole milk or skimmed or partially skimmed milk, condensed milk, powdered milk, lactoserum proteins, whole egg, egg white, egg yolk, soya flour, soya protein concentrates, liquid soya-food preparations, coconut milk, blood serum, meat broth, culture media, in particular media for culturing plant cells or tissues, animal cells or tissues, or media for culturing micro-organisms.

6. Particle according to one of Claims 1 to 5, characterized in that the abovementioned polysaccharide is a gellable polysaccharide, chosen in particular from the group consisting of an alginate, a carrageenan, in particular kappa-carrageenan, a gel able pectin, in particular a low methoxyl pectin, a polysaccharide bearing amino groups which may preferably be gelled at a pH above 6.2 or in the presence of a polyphosphate, such as chitosan.

7. Particle according to one of Claims 1 to 6, characterized in that the abovementioned membrane traps liquid contents consisting of a liquefied gel or of an encapsulated liquid which is aqueous or hydrophobic in nature.

8. Particle according to one of Claims 1 to 6, characterized in that the abovementioned membrane envelops a gelled core comprising either the abovementioned gel able polysaccharide or the abovementioned esterified polysaccharide or alternatively the abovementioned polyamine, in a form which is gelled by a gelling substance.

9. Particle according to one of Claims 1 to 6, characterized in that it consists of a gel which is stiffened throughout its bulk, composed of the product of the so-called transacylation reaction between the esterified polysaccharide and the polyamine optionally having added to them a gellable polysaccharide.

10. Particle according to one of the preceding claims, characterized in that the relative proportion by weight of esterified polysaccharide to the polyamine is between 3% and 500% by weight.

11. Particle according to one of the preceding claims, characterized in that the relative proportion by weight of gellable polysaccharide to the esterified polysaccharide is between 0% and 300% by weight.

12. Particle according to one of the preceding claims, characterized in that it contains one or more active principle(s), in solution, suspension or emulsion form, chosen in particular from the group consisting of a cosmetic or pharmaceutical active principle, a substance of agrifood value, a substance of diagnostic or reagent value, a protein having biological activity such as an enzyme, a hormone, an antibody or haemoglobin, insoluble particles, for example particles of an adsorbent material such as active charcoal, a hydrophobic liquid phase, such as a plant oil, a mineral oil, a silicone oil or an essential oil, or a solution of one or more substances in a hydrophobic liquid phase, a foam containing bubbles of gas such as air, a vaccine for slow release in situ, or living materials such as micro-organisms, cells, tissues or organs from the animal or plant kingdom, or cell constituents such as liver microsomes in order to carry out bioconversions, or genetic material from the animal kingdom or from the plant kingdom.

13. Particle according to Claim 12, characterized in that the abovementioned living materials comprise micro-organisms for performing syntheses or bioconversions, such as bacteria, for instance those used for the fermentations of dairy products, or those used for water purification, or fungi such as mycorrhizas or yeast such as, for example, the yeasts used in the manufacture of beer, or the yeasts used to give champagne its mousse, or microalgae, seeds to which various protective substances or substances influencing their germination or their growth may be added, plant somatic embryos for producing synthetic seeds, plant apices, plant cells or tissues, in particular for carrying out biosyntheses or bioconversions, living material from the animal kingdom, such as cells or tissues which may be used in particular to carry out toxicology tests in vitro, such as liver cells, chondrocytes, neurones, cells or groups of cells or tissues which may be used in the context of a cell therapy, such as the islets of Langerhans for the treatment of diabetes, medullosuprarenal cells or chromaffin cells for the treatment of Parkinson's disease or for the treatment of chronic pains, or cells, which may or may not be genetically modified, which may be used for the production of biological substances such as hormones, enzymes, growth factors, interferon or coagulation factors, or hybridomas for the production of monoclonal antibodies or eggs, gametes, or embryos.

14. Particle according to any one of Claims 1 to 13, characterized in that it has a central part consisting of an aqueous phase which is optionally gelled or liquefied, containing a material, in particular a living material, such as micro-organisms, for instance yeasts or bacteria, or living cells, in particular plant cells or tissues, animal cells or tissues, or groups of cells or tissues, or a biological substance such as, for example, an enzyme, and an external layer not containing the said material and containing, at least at the surface, a membrane formed by the product of the transacylation reaction with formation of covalent amide bonds between at least one esterified polysaccharide and at least one polyamine.

15. Process for the manufacture of particles, in particular beads, capsules, microcapsules, spheres or microspheres, characterized in that it comprises the following successive steps:
a) a first initial aqueous solution which may be gelled by a gelling agent containing at least one esterified polysaccharide and at least one polyamine, which have added to them at least one polysaccharide which is gellable when neither the esterified polysaccharide nor the polyamine are gellable under the operating conditions chosen, is prepared,
b) this first solution is dripped into a second aqueous solution forming a gelling bath, containing a gelling agent, so as to individualize particles by gelation using the said agent,
c) the gelled particles obtained are placed in contact with an alkaline aqueous solution, so as to trigger a so-called transacylation reaction, at least at the surface of the gelled particles, between the esterified polysaccharide and the polyamine which are contained in the said particles, for a predetermined period of time, in order to form a membrane at least at the surface of the said particles,
d) an acidic agent is added to the reaction medium so as to neutralize and thus stabilize the particles, thus obtaining particles comprising, at least at the surface, a membrane formed by the reaction product with formation of covalent bonds between the esterified polysaccharide and the polyamine.

16. Process according to Claim 15, characterized in that the drops of gellable solution are formed by any means for individualizing drops, such as injection systems comprising a syringe fitted with a needle or a peristaltic pump equipped with a tube fitted with a needle, or a system of dispersion by use of a compressed-air vaporizer or alternatively by mechanically cutting, using a vibrator, a laminar flow produced either by extrusion of a solution through a nozzle.

17. Process according to claims 15 for the manufacture of particles, in particular beads, capsules, microcapsules, spheres or microspheres, containing an aqueous or hydrophobic, encapsulated liquid phase, characterized in that it comprises the following successive steps:
a) a first initial aqueous solution which may be gelled by a gelling agent containing at least one esterified polysaccharide and at least one polyamine, which have added to them at least one polysaccharide which is gellable when neither the esterified polysaccharide nor the polyamine are gellable under the operating conditions chosen, is prepared,
b) an aqueous or hydrophobic liquid phase to be encapsulated is provided,
c) a laminar coextrusion of the said first initial aqueous solution as an external flow, and of the aqueous or hydrophobic liquid phase to be encapsulated as an internal flow, is produced through an extrusion nozzle under conditions producing a dissociation of the laminar flow into individual drops, for example by subjecting the laminar flow to vibrations,
d) these drops are dropped into a second aqueous solution forming a gelling bath containing a gelling agent, so as to individualize particles by gelation using the said gelling agent, the said particles containing the said liquid phase to be encapsulated,
e) the gelled particles obtained are placed in contact with an alkaline aqueous solution so as to trigger a so-called transacylation reaction, at least at the surface of the gelled particles, between the esterified polysaccharide and the polyamine which are contained in the said particles, for a predetermined period of time, in order to form a membrane at least at the surface of the said particles,
f) an acidic agent is added to the reaction medium so as to neutralize and thus stabilize the particles, thus obtaining particles comprising, at least at the surface, a membrane formed by the reaction product with formation of covalent bonds between the esterified polysaccharide and the polyamine and containing a drop of the abovementioned liquid to be encapsulated.

18. Process according to claim 17 for the manufacture of particles, in particular beads, capsules, microcapsules, spheres or microspheres, containing a material to be encapsulated which is present exclusively in its central part, characterized in that in step b) an aqueous phase to be encapsulated is prepared, which phase optionally contains a gellable polysaccharide and containing a material, in particular a living material such as micro-organisms, for instance yeasts or bacteria, or living cells or tissues, animals or plants, or a biological substance such as, for example, an enzyme.

19. Process according to one of claims 15 to 18, characterized in that a polyamine is added to the alkalized aqueous solution which is brought into contact with the gelled particles.

20. Process according to one of Claims 15 to 19, characterized in that the gelling agent is a mono- or polyvalent cation, chosen in particular from the group consisting of a calcium salt or a potassium salt, or an iron, aluminum, copper, manganese or barium salt, or an aqueous solution of pH above 6.2 or a polyphosphate solution, depending on the nature of the gelling constituent.

21. Process according to one of Claims 15 to 20, characterized in that the concentration of gelling agent, in particular of mono- or polyvalent cation or of polyphosphate, in the aqueous solution forming the abovementioned gelling bath, is between 0.01 M and 3 M, preferably between 0.8 M and 1 M.

22. Process according to one of Claims 15 to 21, characterized in that the time for which the particles are stirred in the gelling bath is between 1 and 40 minutes.

23. Process according to one of Claims 15 to 22, characterized in that the concentration of abovementioned gellable polysaccharide in the initial aqueous solution to be dispersed and gelled is between 0 and 20%, preferably between 0.5% and 2%, and better still is about 1% by weight/v.

24. Process according to one of Claims 15 to 23, characterized in that the concentration of esterified polysaccharide in the abovementioned initial aqueous solution is between 0.5% and 20% by weight and, further, is preferably in the region of 2% by weight/v.

25. Process according to one of Claims 15 to 24, characterized in that the concentration of polyamine in the abovementioned initial aqueous solution or in the solution in which the gelled particles are dispersed, is between 1% and 30% by weight/v.

26. Process according to one of Claims 15 to 25, characterized in that the alkaline solution triggering the abovementioned transacylation reaction with formation of covalent bonds at least at the surface of the particles, has a pH between 8 and 14 and, further, preferably between 10.5 and 12.5.

27. Process according to Claim 26, characterized in that the alkaline aqueous solution is formed by addition of an alkaline agent in order to bring the pH to the abovementioned pH value, it being possible for this alkaline aqueous solution optionally to be obtained by addition of the alkaline agent to the aqueous solution forming the abovementioned gelling bath.

28. Process according to Claim 27, characterized in that the alkaline agent may be chosen, for example, from sodium hydroxide, potassium hydroxide, aqueous ammonia or an organic amino compound such as, for example, triethanolamine, triethylamine or polyethyleneimine.

29. Process according to one of Claims 15 to 28, characterized in that the time for which the particles are kept in the alkaline solution in order for the transacylation reaction to develop is between 5 min and 1 hour, preferably between 5 min and 30 min and, even more preferably, it is 15 min.

30. Process according to one of Claims 15 to 29, characterized in that the thickness of the membrane, its resistance to enzymatic lysis and the narrowness of its pores may be increased by increasing the duration of the said transacylation reaction and/or by variations in the composition of the alkaline solution triggering the said transacylation reaction and, in particular, by increasing the amount of alkaline agent used to prepare the said alkaline solution.

31. Process according to one of Claims 15 to 30, characterized in that the amount of acidic agent added to the alkaline suspension of particles after the abovementioned transacylation reaction with formation of covalent bonds is such that the water of the particle suspension is neutralized or brought to a slightly acidic pH.

32. Process according to one of Claims 15 to 31, characterized in that the abovementioned acidic agent used to neutralize the alkaline suspension of particles after the so-called transacylation reaction with formation of covalent bonds is, for example, chosen from a monocarboxylic or polycarboxylic organic acid bearing or not bearing an alcohol function, and in particular chosen from the group consisting of acetic acid, citric acid, tartaric acid, succinic acid, malic acid and lactic acid, or an inorganic acid such as hydrochloric acid or sulfuric acid.

33. Process according to one of Claims 15 to 32, characterized in that the time for neutralization of the particles, that is to say the stirring time required after addition of the acid to the reaction medium, is between 5 min and 1 hour, preferably between 5 min and 30 min, and even more preferably it is 15 min.

34. Process according to one of Claims 15 to 33, characterized in that it comprises the additional step of liquefaction of the particle contents, after formation of the abovementioned membrane by the transacylation reaction with formation of covalent bonds, with a liquefying agent.

35. Process according to Claim 34, characterized in that the abovementioned liquefying agent is an aqueous citrate or phosphate solution containing, in particular, a concentration of citrate or of phosphate between 0.01 M and 1 M, preferably between 0.2 M and 0.5 M.

36. Process according to one of Claims 15 to 35, characterized in that, in the initial aqueous solution prepared in the abovementioned step (a) or in the aqueous or hydrophobic liquid phase to be encapsulated by coextrusion, one or more active principles are introduced directly or in solution, suspension or emulsion form, in particular one or more substances of cosmetic, pharmaceutical, biomedical or agrifood value, substances which may be used for diagnosis or as reagents, particles of an adsorbent material such as active charcoal, a foam containing bubbles of gas such as air, a hydrophobic liquid, a vaccine, living materials such as micro-organisms, seeds to which may be added various protective substances or substances influencing their germination or their growth, cells, tissues or organs from the animal kingdom or from the plant kingdom, gametes, embryos or cell constituents.

37. Process according to one of Claims 15 to 36, characterized in that the abovementioned polyamine is a protein endowed with specific biological activity, such as an enzyme, a hormone, an antibody such as a monoclonal antibody, or haemoglobin, alone or mixed with another protein.

38. Process according to one of Claims 15 to 37, characterized in that the particles may be dried, for example by freeze-drying, spraying or in a fluidized bed dryer, for example, with air advantageously heated to a suitable temperature.

39. Composition such as a cosmetic, pharmaceutical, therapeutic, food or enzymatic composition, composition for cell therapy, composition for enzyme therapy, composition for blood purification, composition for reagents, composition for toxicological tests in vitro, compositions for agriculture, compositions for coated seeds, composition for biotechnological production, characterized in that it comprises particles defined in any one of Claims 1 to 14.

## Patentansprüche

1. Teilchen, insbesondere Kügelchen, Kapsel, Mikrokapsel, Sphäre oder Mikrosphäre, dadurch gekennzeichnet, daß es zumindest ein verestertes Polysaccharid und zumindest ein Polyamin umfaßt, denen zumindest ein gelierbares Polysaccharid zugesetzt wird, wenn weder das veresterte Polysaccharid noch das Polyamin unter den gewählten Betriebsbedingungen gelierbar ist, welches Teilchen zumindest an der Oberfläche eine Membran umfaßt, die aus dem Produkt einer Transacylierungsreaktion unter Bildung kovalenter Amid-Bindungen zwischen dem veresterten Polysaccharid und dem Polyamin innerhalb eines gegebenenfalls verflüssigbaren Gels gebildet ist.

2. Teilchen nach Anspruch 1, dadurch gekennzeichnet, daß das veresterte Polysaccharid ein hydrophiles Polysaccharid ist, das Carboxyl-Gruppen trägt, die in einer Menge von zumindest 50 % verestert sind.

3. Teilchen nach einem der Ansprüche 1 und 2, dadurch gekennzeichnet, daß das veresterte Polysaccharid ausgewählt sein kann aus der Gruppe bestehend aus einem veresterten Alginat, insbesondere einem Propylenglykolalginat, einem Pektin, insbesondere einem stark methoxylierten Pektin, oder jeder anderen Verbindung, die durch Veresterung von Carboxylen von Carboxylen tragenden Polysacchariden erhalten werden kann.

4. Teilchen nach einem der Ansprüche 1 bis 3, dadurch gekennzeichnet, daß das Polyamin ein Protein, ein Polypeptid, eine Polyaminosäure, ein Amin-Gruppen tragendes Polysaccharid, wie Chitosan, eine organische, aliphatische, alicyclische oder aromatische Substanz, die mehrere primäre oder sekundäre Amin-Gruppen trägt, wie Ethylendiamin, Pentamethylendiamin, Hexamethylendiamin, Piperazin, Phenylendiamin, Polyethylenimin, umfaßt.

5. Teilchen nach Anspruch 4, dadurch gekennzeichnet, daß das Protein ausgewählt ist aus einem Protein, das hydrophil ist oder behandelt wurde, um hydrophil, d.h. in Wasser löslich oder in Wasser dispergierbar, zu werden, und das freie Amin-Gruppen enthält, vorzugsweise ausgewählt aus der Gruppe bestehend aus Albuminen, wie Serumalbumin, Ovalbumin, α-Lactalbumin, Globulinen, Fibrinogen, Casein, solubilisierten Skleroproteinen, Kollagen, Atelokollagen, Gelatine, Gelatinehydrolysaten, Peptonen, Hämoglobin, Pflanzenproteinen, wie Soja- oder Weizenproteinen, die vorzugsweise abgebaut wurden, Enzymen, wie Katalase, Phosphatasen, Hormonen, Immunglobulinen oder Antikörpern, wie monoklonalen Antikörpern, Polypeptiden, Polyaminosäuren, oder gegebenenfalls natürlichen Mischungen, die ein oder mehrere Proteine umfassen, wie Mischungen von Atelokollagen und Glykosaminoglykanen, Voll- oder vollständig oder teilweise entrahmter Milch, Kondensmilch, Milchpulver, Lactoserumproteinen, ganzem Ei, Eiklar, Eidotter, Sojamehl, Sojaproteinkonzentraten, flüssigen Soja-Nahrungsmittelzubereitungen, Kokosmilch, Blutserum, Kalbsbrühe, Kulturmedien, insbesondere Medien für die Kultur von Pflanzenzellen oder -geweben, Tierzellen oder -geweben oder Medien für die Kultur von Mikroorganismen.

6. Teilchen nach einem der Ansprüche 1 bis 5, dadurch gekennzeichnet, daß das Polysaccharid ein gelierbares Polysaccharid ist, insbesondere ausgewählt aus der Gruppe bestehend aus einem Alginat, einem Carrageenan, insbesondere Kappa-Carrageenan, einem gelierbaren Pektin, insbesondere einem schwach methoxylierten Pektin, einem Polysaccharid, das Amin-Gruppen trägt, und vorzugsweise bei einem pH von mehr als 6,2 oder in Anwesenheit eines Polyphosphats gelierbar ist, wie Chitosan.

7. Teilchen nach einem der Ansprüche 1 bis 6, dadurch gekennzeichnet, daß die Membran einen flüssigen Inhalt einschließt, der aus einem verflüssigten Gel oder einer wässerigen eingekapselten oder hydrophoben Flüssigkeit besteht.

8. Teilchen nach einem der Ansprüche 1 bis 6, dadurch gekennzeichnet, daß die Membran einen gelierten Kern umhüllt, der entweder das gelierbare Polysaccharid oder das veresterte Polysaccharid oder auch das Polyamin in einer in einem Gelierungsgebilde gelierten Form umfaßt.

9. Teilchen nach einem der Ansprüche 1 bis 6, dadurch gekennzeichnet, daß es aus einem in seiner gesamten Masse versteiften Gel besteht, das aus dem Transacylierungsreaktionsprodukt zwischen dem veresterten Polysaccharid und dem Polyamin zusammengesetzt ist, denen gegebenenfalls ein gelierbares Polysaccharid zugesetzt wird.

10. Teilchen nach einem der vorhergehenden Ansprüche, dadurch gekennzeichnet, daß die relative Massemenge des veresterten Polysaccharids in bezug auf das Polyamin zwischen 3 Masse-% und 500 Masse-% beträgt.

11. Teilchen nach einem der vorhergehenden Ansprüche, dadurch gekennzeichnet, daß die relative Massemenge des gelierbaren Polysaccharids in bezug auf das veresterte Polysaccharid zwischen 0 Masse-% und 300 Masse-% beträgt.

12. Teilchen nach einem der vorhergehenden Ansprüche, dadurch gekennzeichnet, daß es einen oder mehrere aktive Bestandteile im Zustand einer Lösung, Suspension oder Emulsion enthält, insbesondere ausgewählt aus der Gruppe bestehend aus einem kosmetischen, pharmazeutischen aktiven Bestandteil, einer Substanz mit agroalimentärem Interesse, einer als Diagnostikum oder Reagenzien interessanten Substanz, einem Protein mit einer biologischen Wirksamkeit, wie einem Enzym, einem Hormon, einem Antikörper oder Hämoglobin, unlöslichen Teilchen, beispielsweise Teilchen eines adsorbierenden Materials, wie Aktivkohle, einer hydrophoben Flüssigphase, wie einem Pflanzenöl, einem Mineralöl, einem Silikonöl, einem etherischen Öl, oder einer Lösung einer oder mehrerer Substanzen in einer hydrophoben Flüssigphase, einem Gasblasen, wie Luft, enthaltenden Schaum, einem Impfstoff zum langsamen Aussalzen in situ, oder lebenden Materialien, wie Mikroorganismen, Zellen, Geweben, Organen, die zum Tier- oder Pflanzenreich gehören, oder Zellbestandteilen, wie Lebermikrosomen zum Bewirken von Bioumwandlungen, oder genetischem Material, das zum Tierreich oder zum Pflanzenreich gehört.

13. Teilchen nach Anspruch 12, dadurch gekennzeichnet, daß die lebenden Materialien umfassen: Mikroorganismen zur Durchführung von Synthesen oder Bioumwandlungen, wie Bakterien, wie Zellen, die für Fermentationen von Milchprodukten verwendet werden, oder Zellen, die zur Entgiftung von Gewässern verwendet werden, oder Pilze, wie Mykorrhizae, oder Hefen, wie beispielsweise Hefen, die bei der Herstellung von Bier verwendet werden, oder Hefen, die zum Moussieren von Champagner verwendet werden, oder Mikroalgen, Samen, denen gegebenenfalls verschiedene Substanzen zugesetzt werden, die ihre Keimung und ihr Wachstum schützen oder beeinflussen, somatische Pflanzenembryonen zur Herstellung von synthetischem Saatgut, Pflanzenspitzen, Pflanzenzellen oder -geweben, insbesondere zum Bewirken von Biosynthesen oder Bioumwandlungen, lebendes Material, das zum Tierreich gehört, wie Zellen oder Gewebe, die insbesondere zur Durchführung von Toxikologietests in vitro verwendet werden können, wie Leberzellen, Chondrocyten, Neuronen, Zellen oder Zellgruppen oder Gewebe, die im Rahmen einer Zelltherapie als Langerhans-Inseln zur Behandlung von Diabetes verwendet werden können, Niebennierenmarkzellen oder chromaffine Zellen zur Behandlung der Parkinson-Krankheit oder zur Behandlung chronischer Schmerzen, oder gegebenenfalls genetisch modifizierte Zellen, die zur Erzeugung biologischer Substanzen verwendet werden können, wie Hormone, Enzyme, Wachstumsfaktoren, Interferon, Koagulationsfaktoren oder Hybridomen zur Erzeugung monoklonaler Antikörper, oder Eizellen, Gameten oder Embryonen.

14. Teilchen nach einem der Ansprüche 1 bis 13, dadurch gekennzeichnet, daß es einen zentralen Teil aufweist, der aus einer gegebenenfalls gelierten oder verflüssigten wässerigen Phase besteht, die ein Material, insbesondere ein lebendes Material, wie Mikroorganismen, wie Hefen oder Bakterien, oder lebende Zellen, insbesondere Pflanzenzellen oder -gewebe, tierische Zellen oder Gewebe, oder Gruppen von Zellen oder Geweben, oder eine biologische Substanz, wie beispielsweise ein Enzym, enthält, und eine äußere Schicht aufweist, die dieses Material nicht enthält und zumindest an der Oberfläche eine Membran umfaßt, die aus dem Transacylierungsreaktionsprodukt unter Bildung kovalenter Amid-Bindungen zwischen zumindest einem veresterten Polysaccharid und zumindest einem Polyamin gebildet ist.

15. Verfahren zur Herstellung von Teilchen, insbesondere Kügelchen, Kapseln, Mikrokapseln, Sphären oder Mikrosphären, dadurch gekennzeichnet, daß es die folgenden aufeinanderfolgenden Schritte umfaßt:
a) eine erste wässerige Ausgangs-Lösung wird hergestellt, die mit einem Gelierungsmittel gelierbar ist, und zumindest ein verestertes Polysaccharid und zumindest ein Polyamin enthält, denen zumindest ein gelierbares Polysaccharid zugesetzt wird, wenn weder das veresterte Polysaccharid noch das Polyamin unter den gewählten Betriebsbedingungen gelierbar ist,
b) Tröpfchen dieser ersten Lösung werden in eine zweite wässerige Lösung fallen gelassen, die ein Gelierungsbad bildet, das ein Geliermittel enthält, um die einzelnen Teilchen durch die Gelierung mit Hilfe des Geliermittels zu trennen,
c) die erhaltenen gelierten Teilchen werden mit einer alkalischen wässerigen Lösung in Berührung gebracht, um eine Transacylierungsreaktion, zumindest an der Oberfläche der gelierten Teilchen, zwischen dem veresterten Polysaccharid und dem Polyamin, die in den Teilchen enthalten sind, während eines vorherbestimmten Zeitraums auszulösen, wobei eine Membran zumindest an der Oberfläche der Teilchen gebildet wird,
d) dem Reaktionsmedium wird ein saures Mittel zugesetzt, um die Teilchen zu neutralisieren und so zu stabilisieren, wobei auf diese Weise Teilchen erhalten werden, welche zumindest an der Oberfläche eine Membran umfassen, die aus dem Reaktionsprodukt unter Bildung kovalenter Bindungen zwischen dem veresterten Polysaccharid und dem Polyamin gebildet wird.

16. Verfahren nach Anspruch 15, dadurch gekennzeichnet, daß die Tröpfchen der gelierbaren Lösung durch jedes Mittel zur einzelnen Auftrennung der Tröpfen gebildet werden, wie Injektionssysteme, die eine Spritze mit einer Nadel oder eine peristaltische Pumpe mit einem mit einer Nadel versehenen Röhrchen oder ein Dispersionssystem unter Verwendung eines Druckluftzerstäubers oder auch durch mechanisches Zerschneiden, mit Hilfe einer Vibrationsvorrichtung, einer laminaren Strömung, die beispielsweise durch die Extrusion einer Lösung durch eine Düse erzeugt wird.

17. Verfahren nach Anspruch 15 zur Herstellung von Teilchen, insbesondere Kügelchen, Kapseln, Mikrokapseln, Sphären oder Mikrosphären, die eine eingekapselte wässerige oder hydrophobe Flüssigphase enthalten, dadurch gekennzeichnet, daß es die folgenden aufeinanderfolgenden Schritte umfaßt:
a) eine erste wässerige Ausgangs-Lösung wird hergestellt, die mit einem Gelierungsmittel gelierbar ist, und zumindest ein verestertes Polysaccharid und zumindest ein Polyamin enthält, denen zumindest ein gelierbares Polysaccharid zugesetzt wird, wenn weder das veresterte Polysaccharid noch das Polyamin unter den gewählten Betriebsbedingungen gelierbar ist,
b) eine wässerige oder hydrophobe Flüssigphase, die einzukapseln ist, wird hergestellt,
c) durch eine Extrusionsdüse wird eine laminare Co-Extrusion der ersten wässerigen Ausgangs-Lösung als äußerer Strömungsschicht und der wässerigen oder hydrophoben Flüssigphase, die einzukapseln ist, als innerer Strömungsschicht unter Bedingungen durchgeführt, die eine Dissoziation der laminaren Strömung in einzelne Tröpfchen bewirken, beispielsweise indem der laminare Strom Vibrationen ausgesetzt wird,
d) diese Tröpfchen werden in eine zweite wässerige Lösung fallen gelassen, die ein Gelierungsbad bildet, das ein Geliermittel enthält, um die einzelnen Teilchen durch die Gelierung mit Hilfe des Geliermittels zu trennen, wobei die Teilchen die einzukapselnde Flüssigphase enthalten,
e) die erhaltenen gelierten Teilchen werden mit einer alkalischen wässerigen Lösung in Berührung gebracht, um eine Transacylierungsreaktion, zumindest an der Oberfläche der gelierten Teilchen, zwischen dem veresterten Polysaccharid und dem Polyamin, die in den Teilchen enthalten sind, während eines vorherbestimmten Zeitraums auszulösen, wobei eine Membran zumindest an der Oberfläche der Teilchen gebildet wird,
f) dem Reaktionsmedium wird ein saures Mittel zugesetzt, um die Teilchen zu neutralisieren und so zu stabilisieren, wobei auf diese Weise Teilchen erhalten werden, welche zumindest an der Oberfläche eine Membran umfassen, die aus dem Reaktionsprodukt unter Bildung kovalenter Bindungen zwischen dem veresterten Polysaccharid und dem Polyamin gebildet wird, und ein Tröpfchen der einzukapselnden Flüssigkeit enthält.

18. Verfahren nach Anspruch 17 zur Herstellung von Teilchen, insbesondere Kügelchen, Kapseln, Mikrokapseln, Sphären oder Mikrosphären, die ein einzukapselndes Material umfassen, das nur in ihrem zentralen Teil vorliegt, dadurch gekennzeichnet, daß in Schritt b) eine einzukapselnde wässerige Phase hergestellt wird, die gegebenenfalls ein gelierbares Polysaccharid umfaßt und ein Material, insbesondere ein lebendes Material, wie Mikroorganismen, wie Hefen oder Bakterien, oder tierische oder pflanzliche lebende Zellen oder Gewebe, oder eine biologische Substanz, wie beispielsweise ein Enzym, enthält.

19. Verfahren nach einem der Ansprüche 15 bis 18, dadurch gekennzeichnet, daß ein Polyamin der alkalisch gemachten wässerigen Lösung zugesetzt wird, die mit den gelierten Teilchen in Berührung gebracht wird.

20. Verfahren nach einem der Ansprüche 15 bis 19, dadurch gekennzeichnet, daß das Geliermittel ein mono- oder polyvalentes Kation ist, insbesondere ausgewählt aus der Gruppe bestehend aus einem Calciumsalz oder einem Kaliumsalz, oder einem Eisen- oder Aluminium- oder Kupfer- oder Mangan- oder Bariumsalz, oder einer wässerigen Lösung mit einem pH von mehr als 6,2 oder einer Polyphosphat-Lösung, gemäß der Beschaffenheit des Gelier-Bestandteils.

21. Verfahren nach einem der Ansprüche 15 bis 20, dadurch gekennzeichnet, daß die Konzentration des Geliermittels, insbesondere des mono- oder polyvalenten Kations oder Polyphosphats in der wässerigen Lösung, die das Gelierbad bildet, zwischen 0,01 M und 3 M, vorzugsweise zwischen 0,8 M und 1 M, liegt.

22. Verfahren nach einem der Ansprüche 15 bis 21, dadurch gekennzeichnet, daß die Zeit, während welcher die Teilchen im Gelierbad gerührt werden, zwischen 1 und 40 Minuten beträgt.

23. Verfahren nach einem der Ansprüche 15 bis 22, dadurch gekennzeichnet, daß die Konzentration des gelierbaren Polysaccharids in der zu dispergierenden und gelierenden wässerigen Ausgangs-Lösung zwischen 0 und 20 %, vorzugsweise zwischen 0,5 % und 2 %, bevorzugt etwa 1 %, G/V beträgt.

24. Verfahren nach einem der Ansprüche 15 bis 23, dadurch gekennzeichnet, daß die Konzentration des veresterten Polysaccharids in der wässerigen Ausgangs-Lösung zwischen 0,5 Masse-% und 20 Masse-% liegt, und vorzugsweise etwa 2 % G/V beträgt.

25. Verfahren nach einem der Ansprüche 15 bis 24, dadurch gekennzeichnet, daß die Konzentration des Polyamins in der wässerigen Ausgangs-Lösung oder in der Lösung, in der die gelierten Teilchen dispergiert werden, zwischen 1 % und 30 % G/V beträgt.

26. Verfahren nach einem der Ansprüche 15 bis 25, dadurch gekennzeichnet, daß die alkalische Lösung, welche die Transacylierungsreaktion unter Bildung kovalenter Bindungen zumindest an der Oberfläche der vorliegenden Teilchen auslöst, einen pH zwischen 8 und 14, vorzugsweise zwischen 10,5 und 12,5, aufweist.

27. Verfahren nach Anspruch 26, dadurch gekennzeichnet, daß die alkalische wässerige Lösung durch den Zusatz eines alkalischen Mittels gebildet wird, um den pH auf den angegebenen pH-Wert zu führen, wobei diese alkalische wässerige Lösung gegebenenfalls durch den Zusatz eines alkalischen Mittels in der wässerigen Lösung gebildet werden kann, die das Gelierbad bildet.

28. Verfahren nach Anspruch 27, dadurch gekennzeichnet, daß das alkalische Mittel beispielsweise ausgewählt werden kann aus Soda, Kali, Ammoniak oder einer organischen Amin-Verbindung, wie beispielsweise Triethanolamin, Triethylamin, Polyethylenimin.

29. Verfahren nach einem der Ansprüche 15 bis 28, dadurch gekennzeichnet, daß die Zeit, während welcher die Teilchen innerhalb der alkalischen Lösung gehalten werden, damit sich die Transacylierungsreaktion entwickelt, zwischen 5 Minuten und 1 Stunde, vorzugsweise zwischen 5 Minuten und 30 Minuten liegt, und bevorzugt 15 Minuten beträgt.

30. Verfahren nach einem der Ansprüche 15 bis 29, dadurch gekennzeichnet, daß die Dicke der Membran, ihre Beständigkeit gegenüber einer enzymatischen Lyse und die Enge ihrer Poren durch die Erhöhung der Dauer der Transacylierungsreaktion und/oder durch Variationen der Zusammensetzung der alkalischen Lösung, welche die Transacylierungsreaktion auslöst, und insbesondere durch die Erhöhung der zur Herstellung der alkalischen Lösung verwendeten Menge des alkalischen Mittels erhöht werden kann.

31. Verfahren nach einem der Ansprüche 15 bis 30, dadurch gekennzeichnet, daß die Menge des sauren Mittels, das der alkalischen Teilchen-Suspension nach der Transacylierungsreaktion unter Bildung kovalenter Bindungen zugesetzt wird, derart ist, daß das Wasser der Teilchen-Suspension neutralisiert oder auf einen leicht sauren pH geführt wird.

32. Verfahren nach einem der Ansprüche 15 bis 31, dadurch gekennzeichnet, daß das saure Mittel, das zur Neutralisation der alkalischen Teilchen-Suspension nach der Transacylierungsreaktion unter Bildung kovalenter Bindungen verwendet wird, beispielsweise ausgewählt wird aus einer organischen Monocarbon- oder Polycarbonsäure, die gegebenenfalls eine Alkohol-Funktion trägt, und insbesondere ausgewählt wird aus der Gruppe bestehend aus Essigsäure, Citronensäure, Weinsäure, Bernsteinsäure, Äpfelsäure, Milchsäure oder einer Mineralsäure, wie Salzsäure oder Schwefelsäure.

33. Verfahren nach einem der Ansprüche 15 bis 32, dadurch gekennzeichnet, daß die Zeit der Neutralisation der Teilchen, d.h. die erforderliche Zeit des Rührens nach dem Zusatz der Säure zum Reaktionsmedium, zwischen 5 Minuten und 1 Stunde, vorzugsweise zwischen 5 Minuten und 30 Minuten liegt, und bevorzugt 15 Minuten beträgt.

34. Verfahren nach einem der Ansprüche 15 bis 33, dadurch gekennzeichnet, daß es den ergänzenden Schritt des Verflüssigens des Inhalts der Teilchen nach der Bildung der Membran durch die Transacylierungsreaktion unter Bildung kovalenter Bindungen mit einem Verflüssigungsmittel umfaßt.

35. Verfahren nach Anspruch 34, dadurch gekennzeichnet, daß das Verflüssigungsmittel eine wässerige Citrat- oder Phosphat-Lösung ist, die insbesondere eine Citrat- oder Phosphat-Konzentration zwischen 0,01 M und 1 M und vorzugsweise zwischen 0,2 M und 0,5 M enthält.

36. Verfahren nach einem der Ansprüche 15 bis 35, dadurch gekennzeichnet, daß in die wässerige Ausgangs-Lösung, die in Schritt a) hergestellt wird, oder in die wässerige oder hydrophobe Flüssigphase, die durch Co-Extrusion einzukapseln ist, ein oder mehrere aktive Bestandteile direkt oder im Lösungs-, Suspensions- oder Emulsionszustand eingebracht werden, insbesondere eine oder mehrere Substanzen mit kosmetischem, pharmazeutischen, biomedizinischen oder agroalimentärem Interesse, Substanzen, die als Diagnostikum oder als Reagenzien verwendet werden können, Teilchen eines adsorbierenden Materials, wie Aktivkohle, ein Schaum, der Gasblasen, wie Luft, enthält, eine hydrophobe Flüssigkeit, ein Impfstoff, lebende Materialien, wie Mikroorganismen, Samen, denen gegebenenfalls verschiedene Substanzen zugesetzt werden, die ihre Keimung oder ihr Wachstum schützen oder beeinflussen, Zellen, Gewebe oder Organe des Tierreichs oder des Pflanzenreichs, Gameten, Embryonen, Zellbestandteile.

37. Verfahren nach einem der Ansprüche 15 bis 36, dadurch gekennzeichnet, daß das Polyamin ein Protein mit einer spezifischen biologischen Wirksamkeit ist, wie ein Enzym, ein Hormon, ein Antikörper, wie ein monoklonaler Antikörper, Hämoglobin, allein oder mit einem anderen Protein gemischt.

38. Verfahren nach einem der Ansprüche 15 bis 37, dadurch gekennzeichnet, daß die Teilchen getrocknet werden können, beispielsweise durch Gefriertrocknung, Vernebelung, oder in einem Fließbett-Trockner, beispielsweise mit auf eine geeignete Temperatur vorgeheizter Luft.

39. Zusammensetzung, wie eine kosmetische, pharmazeutische, therapeutische, alimentäre, enzymatische Zusammensetzung, Zusammensetzung zur Zelltherapie, Zusammensetzung zur Enzymtherapie, Zusammensetzung zur Blutreinigung, Zusammensetzung für Reagenzien, Zusammensetzung für toxikologische Tests in vitro, Zusammensetzungen für die Landwirtschaft, Zusammensetzungen für beschichtete Samen, Zusammensetzung zur biotechnologischen Produktion, dadurch gekennzeichnet, daß sie in einem der Ansprüche 1 bis 14 definierte Teilchen umfaßt.
